# EUROPEAN PATENT APPLICATION

(11) **EP 3 831 349 A1**
(43) Date of publication of application: **09.06.2021**
(21) Application number: 21151817.0
(22) Date of filing: 13.06.2013
(51) Int. Cl.: A61F 13/02, A61L 27/58, A61L 27/54, A61F 5/08, A61F 2/18

(54) **BIODEGRADABLE, ACTIVE INGREDIENT-ELUTING STRUCTURAL SUPPORT**

(30) Priority: 13.06.2012 US 201261659357 P; 16.11.2012 US 201213679978
(62) Divisional of application: 13804069.6
(71) Applicant: Matheny Enterprises, LLC, Plano, Texas 75093 (US)
(72) Inventor: MATHENY, Keith E., Plano, Texas 75093 (US)
(74) Representative: Paulraj, Leonita Theresa

(57) **Abstract**

A biodegradable nasal support configured for placement within a paranasal sinus or an opening to the paranasal sinus of a patient, the nasal support comprising a tubular structure having a tubular wall at least partially defining an interior lumen, wherein the biodegradable nasal support consists essentially of a degradable material and, optionally, an active ingredient, the degradable material comprising chitosan and a polymer comprising acrylic acid monomers.

## Description

### BACKGROUND

There exists a trend in the art and science of medicine to continually refine the ability to precisely deliver current and future therapies, be they conventional pharmaceuticals, gene therapeutics, or nanotechnologically-derived compounds, to the location within the patient where they are required. At the same time, equally desirable is precision in minimizing the collateral damage or "side effects" incurred by these agents as they travel elsewhere within the body where they are not therapeutically necessary, or worse, where they may be harmful or toxic. Many conventional, current medicines just narrowly tip the balance in favor of therapeutic benefit when weighed against the risks and side effects, either at the intended site of treatment or elsewhere within the body. For example, in the context of oncology, chemotherapeutic agents are utilized to kill malignant tumor cells at a slightly faster rate than they kill normal cells vital to the survival and health of the patient.

Conventional, relatively-localized therapies, include topical creams and ointments for dermatologic conditions, aerosolized nasal sprays for allergic rhinitis, and nebulized medications for upper and lower airway reactive inflammatory diseases such as chronic rhinosinusitis and asthma. While such therapeutics represent an improvement over non-discriminatory, systemically-administered therapeutics such as corticosteroids, which may have harsh potential side effect profiles, such therapeutics are limited in local therapeutic benefit while still resulting in local, regional, and/or distant toxicity.

Additionally, many current medical therapies and/or therapeutic devices are beneficial for a finite, well-defined period of time, but they, or their delivery vehicles, can outlast their usefulness within the body, and become potentially harmful the longer they remain within the patient.

As such, there is a need for improved means of medical therapies and/or therapeutic devices, for example, that will allow therapeutics to be delivered locally, while subsisting for a desired, beneficial duration of time.

### SUMMARY

Disclosed herein is a biodegradable, active ingredient-eluting structural support and methods of making and using the same.

Disclosed herein is a biodegradable nasal splint, wherein the biodegradable nasal splint is configured for placement within a nasal passage so as to stabilize a surgically-corrected septum.

Also disclosed herein is a biodegradable nasal splint, wherein the biodegradable nasal splint is configured for placement within a nasal passage so as to exert a lateralizing force to an outfractured inferior turbinate.

Further disclosed herein is a method comprising performing a corrective procedure with respect to a patient's nasal passage, positioning a biodegradable nasal splint within the patient's nasal passage, wherein the biodegradable nasal splint is effective to exert a lateralizing force to an inferior turbinate, to stabilize a nasal septum, or combinations thereof.

### BRIEF DESCRIPTION OF THE DRAWINGS

For a more complete understanding of the present disclosure and the advantages thereof, reference is now made to the following brief description, taken in connection with the accompanying drawings and detailed description:
Figure 1 is an illustration of an embodiment of a biodegradable, active ingredient-eluting structural support and/or a biodegradable structural support having a cylindrical configuration;
Figure 2 is an illustration of an embodiment of a biodegradable, active ingredient-eluting structural support and/or a biodegradable structural support having a cylindrical configuration;
Figure 3 is an illustration of an embodiment of a biodegradable, active ingredient-eluting structural support and/or a biodegradable structural support having a cylindrical configuration;
Figure 4 is an illustration of an embodiment of a biodegradable, active ingredient-eluting structural support and/or a biodegradable structural support having a cylindrical configuration;
Figure 5 is an illustration of an embodiment of a biodegradable, active ingredient-eluting structural support and/or a biodegradable structural support having a cylindrical configuration;
Figure 6 is an illustration of an embodiment of a biodegradable, active ingredient-eluting structural support and/or a biodegradable structural support having a sheet-like configuration;
Figure 7 is an illustration of an embodiment of a biodegradable, active ingredient-eluting structural support and/or a biodegradable structural support having a sheet-like configuration;
Figure 8A is an illustration of an embodiment of a biodegradable, active ingredient-eluting structural support and/or a biodegradable structural support configured as a nasal splint;
Figure 8B is an illustration of an embodiment of a biodegradable, active ingredient-eluting structural support and/or a biodegradable structural support comprising a nasal splint deployed within the nasal passages;
Figure 8C is an illustration of an embodiment of a biodegradable, active ingredient-eluting structural support and/or a biodegradable structural support configured as a nasal splint;
Figure 8D is an illustration of an embodiment of a biodegradable, active ingredient-eluting structural support and/or a biodegradable structural support configured as a nasal splint;
Figure 9A is an illustration of an embodiment of a biodegradable, active ingredient-eluting structural support and/or a biodegradable structural support configured as a nasal splint;
Figure 9B is an illustration of an embodiment of a biodegradable, active ingredient-eluting structural support and/or a biodegradable structural support comprising a nasal splint deployed within the nasal passages;
Figure 10A is an illustration of an embodiment of a biodegradable, active ingredient-eluting structural support and/or a biodegradable structural support configured as an over-the-counter nasal splint;
Figure 10B is an illustration of an embodiment of a biodegradable, active ingredient-eluting structural support and/or a biodegradable structural support configured as an over-the-counter nasal splint;
Figure 10C is an illustration of an embodiment of a biodegradable, active ingredient-eluting structural support and/or a biodegradable structural support comprising an over-the-counter nasal splint deployed within the nasal passages;
Figure 10D is an illustration of an embodiment of a biodegradable, active ingredient-eluting structural support and/or a biodegradable structural support comprising an over-the-counter nasal splint deployed within the nasal passages;
Figure 11A is an illustration of an embodiment of a biodegradable, active ingredient-eluting structural support and/or a biodegradable structural support configured as a myringotomy tube;
Figure 11B is an illustration of an embodiment of a biodegradable, active ingredient-eluting structural support and/or a biodegradable structural support configured as a myringotomy tube;
Figure 11C is an illustration of an embodiment of a biodegradable, active ingredient-eluting structural support and/or a biodegradable structural support comprising a myringotomy tube deployed within the tympanic membrane;
Figure 12 is an illustration of an embodiment of a biodegradable, active ingredient-eluting structural support and/or a biodegradable structural support having a multi-use sheet;
Figure 13A is an illustration of an embodiment of a biodegradable, active ingredient-eluting structural support and/or a biodegradable structural support configured as an intrauterine device;
Figure 13B is an illustration of an embodiment of a biodegradable, active ingredient-eluting structural support and/or a biodegradable structural support comprising an intrauterine device deployed within the uterus;
Figure 14A is an illustration of an embodiment of a biodegradable, active ingredient-eluting structural support and/or a biodegradable structural support configured as an constrictive device;
Figure 14B is an illustration of an embodiment of a biodegradable, active ingredient-eluting structural support and/or a biodegradable structural support configured as an constrictive device;
Figure 14C is an illustration of an embodiment of a biodegradable, active ingredient-eluting structural support and/or a biodegradable structural support configured as a constrictive device; and
Figure 14D is an illustration of an embodiment of a biodegradable, active ingredient-eluting structural support and/or a biodegradable structural support comprising a constrictive device deployed at least partially around the esophagus.

### DETAILED DESCRIPTION OF THE EMBODIMENTS

In the drawings and description that follow, like parts are typically marked throughout the specification and drawings with the same reference numerals, respectively. The drawing figures are not necessarily to scale. Certain features of the invention may be shown exaggerated in scale or in somewhat schematic form and some details of conventional elements may not be shown in the interest of clarity and conciseness. The present invention is susceptible to embodiments of different forms. Specific embodiments are described in detail and are shown in the drawings, with the understanding that the present disclosure is not intended to limit the invention to the embodiments illustrated and described herein. It is to be fully recognized that the different teachings of the embodiments discussed herein may be employed separately or in any suitable combination to produce desired results.

Unless otherwise specified, use of the terms "connect," engage," "couple," attach," or any other like term describing an interaction between two or more elements is not meant to limit the interaction to direct interaction between the elements and may also include indirect interaction between the elements described.

Disclosed herein are embodiments of devices, apparatuses, systems, and methods associated with a biodegradable structural support (BSS) and/or a biodegradable, active ingredient-eluting structural support (BAIESS). In one or more of the embodiments as will be disclosed herein, the BAIESS may be configured to provide a structural support at a site of deployment and/or, at least partially contemporaneously therewith, to provide the active ingredient (AI) at the site of BAIESS deployment and/or proximate thereto. Additionally or alternatively, in an embodiment as will be disclosed herein, an AI may be absent; for example, in an embodiment the BSS may be configured to provide a structural support at a site of deployment while not eluting an AI. It is therefore understood that reference, in any one or more of the embodiments disclosed herein, to a "BSS" and/or to a "BAIESS" should be construed as collectively including embodiments comprising an AI as well as embodiments where an AI is absent, including but not limited to embodiments where other, non-active ingredients, additives, or the like may or may not be present.

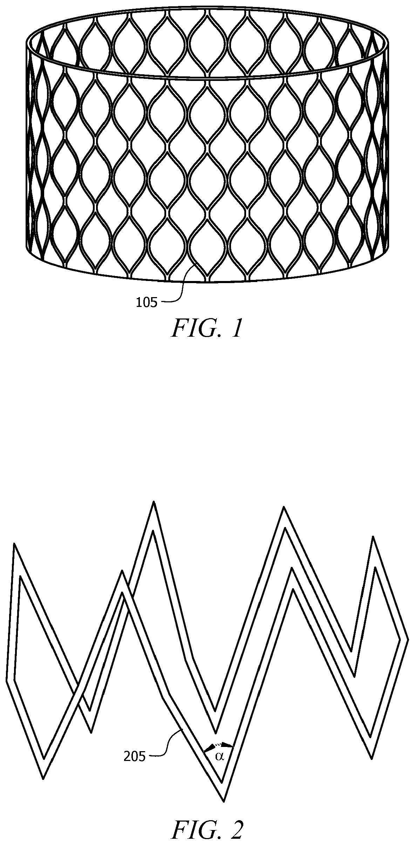

Referring to Figures 1 through 15, embodiments of a BSS and/or BAIESS are illustrated. In one or more of the illustrated embodiments, the BSS/BAIESS generally comprises a platform, for example, a structurally or mechanically active platform, which may or may not have one or more AIs applied thereto.

In an embodiment, the platform generally comprises a structural component. As will also be disclosed herein, the platform may also be configured to apply a physical influence (e.g., a force) to a bodily tissue, as will be disclosed herein, for example, so as to hold open a passageway, cavity, or orifice, to constrict a passageway or cavity, to retain bodily tissue(s) together and/or in place, to inhibit constriction of a passageway or orifice, to promote constriction of a passageway, to maintain ventilation or free exchange of fluids into and/or out of one or more physiologic chambers, to physically impede a pathogen or a fertilized egg access to a tissue, to prevent an unfertilized egg to travel from the ovary to the uterus via the Fallopian tubes, to prevent sperm from traveling from the testes into the eventual semen, to promote tissue healing, or to provide hemostasis or prevent thrombosis, or combinations thereof. Also, in an embodiment where an AI is present as will be disclosed herein, the platform may be configured to provide a structure for the attachment, retention, delivery, and/or elution of the AI.

In an embodiment, the platform may be formed from a suitable component, combinations of operably-coupled components, material(s), or combinations thereof. For example, in an embodiment, the platform may be formed from one or more strands. For example, in an embodiment, a plurality of strands is assembled into a structure that forms all or a portion of the platform. For example, such a plurality of strands may be assembled, connected, or interrelated to form simple or complex geometrical structures or forms. In such an embodiment, such strands may take the form of filaments, ribbons, tapes, bands, threads, or the like. Such strands may comprise a suitable cross-sectional shape. For example, the strands may have a substantially flat, round, oval, square, triangular, rectangular, tear-drop-shaped, diamond, hollow, or other suitable shape or cross-section, or combinations thereof.

In such an embodiment, such a strand or strands may be characterized as having a suitable size, for example, thickness and/or length. For example, in an embodiment, the strands may have a thickness in the range of from about 0.5 mm to about 5 mm, alternatively, from about 1mm to about 4mm, alternatively, from about 2mm to about 3 mm. Also, in an embodiment, the strands may have a length that ranges dependent upon the intended use of the BSS/BAIESS, the configuration of the platform, processing parameters associated with making and/or use the platform and/or the BSS/BAIESS, or combinations thereof, as will be disclosed herein.

In an embodiment, the one or more strands may be operably coupled to form the platform and/or a portion thereof. For example, in the embodiment of Figure 1, each of the plurality of strands 105 (alternatively, a single strand which has been folded or doubled back-and-forth) comprises a generally undulating (e.g., sinusoidal in appearance) configuration, for example having a plurality of "peaks" and "troughs." As used herein, the terms "peaks" and "troughs" are used to refer to the alternating points of inflection of the undulating strands 105, but are not to be construed as denoting any particular absolute orientation (e.g., up or down) of such strands. In the embodiment of Figure 1, the strands 105 are positioned such that the long axis of each of the strands 105 are substantially parallel and offset such that the peak of a given strand may be joined to the trough of an adjacent strand 105. In an embodiment, the adjacent strands may be joined, connected, or interrelated (e.g., fused, adhered, or the like) by any suitable means; in an alternative embodiment, the platform may be formed such that adjacent strands comprise a unitary structure.

Alternatively, in the embodiment of Figure 2, each of the plurality of strands 205 (alternatively, a single strand which has been folded or doubled back-and-forth), comprises a strut in the form of a helical segment (e.g., a portion of a helix). In the embodiment of Figure 2, each of the strands (e.g., struts) is joined to another (e.g., an adjacent) strand (e.g., strut) at the terminal ends thereof at a suitable angle, α. Such a suitable angle, α, may be in the range of from about 10 degrees to about 140 degrees, alternatively, from about 30 degrees to about 130 degrees. As noted above, in such an embodiment, the adjacent strands may be joined (e.g., fused, adhered, or the like) by any suitable means; in an alternative embodiment, the platform may be formed such that adjacent strands comprise a unitary structure.

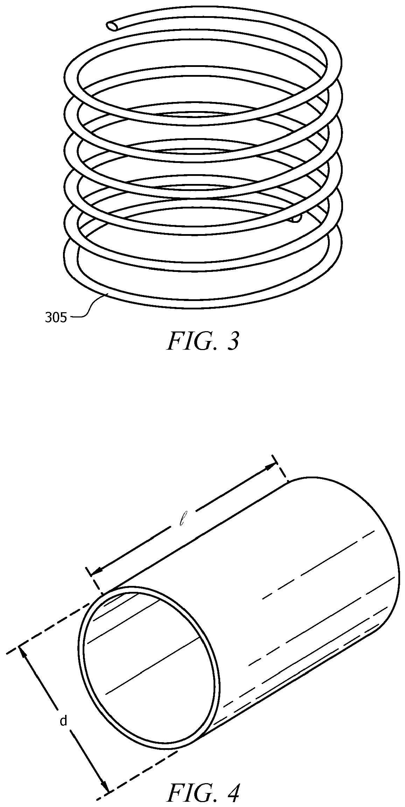

In another embodiment, the platform may comprise a single strand. For example, in the embodiment of Figure 3, the platform is formed from a strand 305 (e.g., a continuous strand) in the shape of a helix. In an embodiment, such a helical strand may exhibit any suitable pitch and/or helical angle. Additionally, such a helical strand may exhibit a constant pitch and/or helical angle; alternatively, such a helical strand may exhibit a variable pitch and/or helical angle.

In yet another embodiment, a plurality of strands may be woven and/or interlaced together. Such a plurality of strands may be woven and/or interlaced at any suitable spacing, in any suitable pattern, and/or at any suitable angle. In an embodiment, interwoven and/or interlaced strands (e.g., weft strands and warp strands) may be fused and/or adhered at the union between any two or more strands. In an embodiment, the strands may be woven to a mesh, a cloth, or other woven material.

In an alternative embodiment, the platform may be formed from a sheet, a film, membrane, or the like. For example, such a sheet, film, or membrane may generally comprise a thickness of platform-forming material (e.g., as will be disclosed herein) in a solid, partially solid, porous, or other suitable configuration. In an embodiment, such a sheet may be characterized as having a suitable size, for example, thickness, width and/or length. For example, in an embodiment, the sheet may have a thickness in the range of from about 0.5 mm to about 5 mm, alternatively, from about 1 mm to about 4 mm, alternatively, from about 2 mm to about 4 mm. Also, in an embodiment, the sheet may have a width and/or length that ranges dependent upon the intended use of the BSS/BAIESS, the configuration of the platform, or combinations thereof, processing parameters associated with making and/or use the platform and/or the BSS/BAIESS, or combinations thereof, as will be disclosed herein. In an embodiment, a sheet may be utilized (e.g., to form the BSS/BAIESS) in a single layer; alternatively, in an embodiment, a sheet may be utilized (e.g., to form the BSS/BAIESS) in multiple layers.

In another alternative embodiment, the platform may comprise a molded component. For example, the platform may be molded and/or otherwise formed, as will be disclosed herein, into any suitable shape or conformation.

In an embodiment, the platform and/or the components and/or materials utilized to form the platform may be characterized as biodegradable. As used herein, the term "biodegradable" and/or like terms may refer to a physical component, or a portion thereof, that is configured to degrade and/or biodegrade. As used herein, the terms "degrade," "biodegrade," and/or like terms may be used interchangeably and may refer to an irreversible process by which a material is substantially and/or completed removed. For example, upon degradation and/or biodegradation, at least a portion of the volume associated with such a material may dissolve, dissipate, or the like, over a duration of time upon deployment in a biological environment (for example, upon deployment so as provide contact with a bodily fluid, such as a mucosal membrane; a bodily tissue, such as connective, muscular, nervous, and/or epithelial tissue; or combinations thereof). In an embodiment, a material (e.g., or the platform or a component thereof) which is degraded and/or biodegraded may be absorbed by a tissue, adsorbed by a tissue, resorbed by a tissue, dissipated and/or carried away from and/or out the body, or combinations thereof. For example, in an embodiment the platform may be configured to biodegrade upon placement within the human body (e.g., *in vivo),* for example, as will be disclosed herein.

In an embodiment, the platform may be configured to degrade, for example, to a suitable extent, over a suitable period of time, for example, as may be at least partially dependent upon the intended use of the platform and/or the BSS/BAIESS. For example, in an embodiment the platform may be configured such that at least 50%, alternatively, at least 60%, alternatively, at least 70%, alternatively, at least 80%, alternatively, at least 90%, alternatively, at least 95%, alternatively, at least 97.5%, alternatively, at least 98%, alternatively, at least 99%, alternatively, at least 99.5%, alternatively, at least 99.9% of the volume associated with the components and/or materials utilized to form the platform may degrade and/or biodegrade within a suitable duration of time. For example, in an embodiment, the components and/or materials utilized to form the platform may degrade and/or biodegrade to a such a desired extent in a duration ranging from (A) a start point of not less than about 72 hours, alternatively, not less than about 96 hours, alternatively, not less than about 5 days, alternatively not less than about 7 days, alternatively, not less than about 14 days, alternatively, not less than about 21 days, alternatively, not less than about 28 days, alternatively, not less than about 1 month, alternatively, not less than about 2 months, alternatively, not less than about 3 months, alternatively, not less than about 4 months, alternatively, not less than about 6 months, alternatively, not less than about 8 months, alternatively, not less than about 10 months, alternatively, not less than about 12 months to (B) an end point of not more than about 7 days, alternatively, not more than about 14 days, alternatively, not more than about 21 days, alternatively, not more than about 28 days, alternatively, not more than about 1 month, alternatively, not more than about 2 months, alternatively, not more than about 3 months, alternatively, not more than about 4 months, alternatively, not more than about 6 months, alternatively, not more than about 8 months, alternatively, not more than about 10 months, alternatively, not more than about 12 months, alternatively, not more than about 15 months, alternatively, not more than about 18 months, alternatively, not more than about 24 months. Reference herein to a particular rate and/or duration of degradation associated with a BSS/BAIESS should not be construed as indicating that such BSS/BAIESS must be placed (e.g., *in vivo)* in order to ascertain such rate and/or duration. For example, as will be appreciated by one or skill in the art upon viewing this disclosure, a rate and/or duration of degradation may be estimated, ascertained, and/or estimated via any suitable protocol. For example, a rate and/or duration of degradation may obtained utilizing a suitable test protocol designed to mimic in vivo conditions at and/or proximate to the intended site of deployment, as will be disclosed herein and, for example, taking into consideration factors including, but not necessarily limited to, the intended site of deployment, ambient conditions at the intended site of deployment (e.g., temperature, moisture, pH, physical interaction with tissue(s), airflow), or combinations thereof.

In an embodiment, the platform may be configured to degrade at a desired rate. For example, in an embodiment the platform may be configured to degrade at a substantially constant rate. In an alternative embodiment, the platform may be configured to degrade at a rate that varies over time. For example, in an embodiment the platform may degrade at a generally increasing rate; alternatively, in an embodiment the platform may degrade at a generally decreasing rate. Additionally, in an embodiment, a first component of the platform may degrade at a first rate while a second component thereof may degrade at a second rate. For example, a first component may be entirely or substantially degraded while a second component is less than 90%, alternatively, less than 80%, alternatively, less than 70%, alternatively, less than 60%, alternatively, less than 50%, alternatively, less than 40%, alternatively, less than 30%, alternatively, less than 20% degraded. As such, the structure of the platform may be configured to change over the course of the degradation and/or biodegradation.

For example, in an embodiment, the BSS/BAIESS may be configured such that the rate of degradation of the platform varies with respect to time. For example, in an embodiment, the platform may comprise a single degradable material, as will be disclosed herein, that is degraded at a rate that varies over time. In an embodiment, multiple degradable materials (having differing rates of degradation) may form the platform such that the platform may be degraded at a rate that varies. For example, one or more degradable materials may form in a plurality of layers, together forming the platform (e.g., a first layer of a first degradable material and a second layer of a second degradable material).

Additionally, in an embodiment the platform may be configured such that the rate of degradation may be altered via the addition or subtraction of a degradation-rate modifying agent. For example, in an embodiment, the platform may be configured to exhibit a first (e.g., relatively slow) rate of degradation prior to exposure to and/or contact with a degradation accelerator and to exhibit a second (e.g., relatively fast) rate of degradation upon exposure to and/or contact with such a degradation accelerator. In such an embodiment, the degradation accelerator may comprise an enzymatically-active solution, an irrigating solution (e.g., a hypotonic) saline solution, ultra-violet (UV) radiation, sonic pulsation, the like, or combinations thereof. For example, addition of and/or exposure to such a degradation accelerator may have the effect of causing the platform to rapidly degrade or "self-destruct." Alternatively, in an embodiment, the platform may be configured to exhibit a first (e.g., relatively slow) rate of degradation while exposed to and/or in contact (e.g., either constantly or intermittently) with a degradation retarder and to exhibit a second (e.g., relatively fast) rate of degradation upon ceasing to be exposed to and/or in contact with such a degradation retarder. For example, removal of and/or ceased exposure to such a degradation may have the effect of causing the platform to rapidly degrade or self-destruct. Additionally, in an embodiment such a degradation-rate modifying agent (e.g., accelerator and/or retarder) may be included within the platform. For example, the degradation-rate modifying agent may be encapsulated, microencapsulated, or the like, for example, such that the capsules/microcapsules may burst, rupture, or otherwise be caused to release the degradation-rate modifying agent upon a suitable stimulus.

Not intending to be bound by theory, the rate, duration, and/or extent of degradation of the BSS/BAIESS, the platform forming the BSS/BAIESS, and/or the materials and/or components forming the platform may be at least partially dependent upon the composition of such materials and/or components, the sizing (e.g., thickness) of such materials and/or components, environmental factors, as will be disclosed herein, or combinations thereof.

In an embodiment, the platform may be configured such that the degradation or biodegradation of the platform and/or the components thereof may be monitored, for example, *in situ.* For example, in an embodiment, the platform (e.g., the composition utilized to form the platform and/or components thereof) may comprise a contrast media. Examples of such a contrast media include, but are not limited to, a radio-contrast agent (e.g., a radio-opaque or substantially radio-opaque material, such as iodine and/or barium), a magnetic resonance imaging (MRI) contrast agent (e.g., a gadolinium-containing compound, an iron-oxide-containing compound, iron platinum particles, a manganese-containing compound), an ultrasound-enhancing material, or combinations thereof. In an embodiment, such a contrast media may be detected (e.g., *in vivo)* and utilized to determine the amount of the platform (or a component thereof) remaining within the body, for example, by comparison to a scale or other standard associated with the degradable material.

In an embodiment and not intending to be limited by theory, the platform may degrade via a surface erosion mechanism characterized by a layer by layer degradation of the platform. Alternatively, the platform may degrade via bulk erosion characterized by erosion occurring throughout the platform. Also not intending to be bound by theory, the platform may degrade by any suitable mode of action. Modes of degradation may be affected through the use of external stimuli such as temperature, light, or heat. Alternatively, degradation of the polymer may occur through contact with one or more materials that facilitate chemical degradation of the polymer. Without limitation the mechanisms of polymer degradation may involve hydrolysis, oxidation, aminolysis, enzymatic degradation (e.g., proteolytic degradation), physical degradation, or combinations thereof.

In an embodiment, the components and/or materials (e.g., as may be utilized to form the BSS/BAIESS) may be formed from any suitable composition, for example, a biodegradable composition, as disclosed herein. For example, in an embodiment, the components and/or materials utilized to form the BSS/BAIESS may comprise one or more polymers, particularly, one or more degradable (e.g., biodegradable) polymers. In various embodiments, the selection of the polymer and/or the combination of polymers utilized may be dependent, at least in part, upon the intended use of the BSS/BAIESS; the AI employed with the BAIESS; the desired rate, duration, and/or extent of biodegradation; or combinations thereof.

In an embodiment, the polymer may be present within the components and/or materials utilized to form the BSS/BAIESS in a range of at least about 5%, at least about 10%, at least about 20%, at least about 30%, at least about 40%, at least about 50%, at least about 60%, at least about 70%, at least about 80%, at least about 90%, at least about 95%, or at least about 100% by total weight of such components and/or materials.

Examples of a polymer (e.g., a degradable polymer) that may be suitable for use in making the components and/or materials which may form the BSS/BAIESS include, but are not limited to, a poly(lactide); a poly(glycolide); a poly(lactide-co-glycolide); a poly(lactic acid); a poly(glycolic acid); a poly(lactic acid-co-glycolic acid); poly(lactide)/poly(ethylene glycol) copolymers; a poly(glycolide)/poly(ethylene glycol) copolymer; a polyhydroxy-alkanoate, a poly(lactide-co-glycolide)/poly(ethylene glycol) copolymer; a poly(lactic acid)/poly(ethylene glycol) copolymer; a poly(glycolic acid)/poly(ethylene glycol) copolymer; a poly(lactic acid-co-glycolic acid)/poly(ethylene glycol) copolymer; a poly(caprolactone); poly(caprolactone)/poly(ethylene glycol) copolymer; a poly(orthoester); a poly(phosphazene); a poly(hydroxybutyrate) or a copolymer including a poly(hydroxybutyrate); a poly(lactide-co-caprolactone); a polycarbonate; a polyesteramide; a polyanhidride; a poly(dioxanone); a poly(alkylene alkylate); a copolymer of polyethylene glycol and a polyorthoester; a biodegradable polyurethane; a poly(amino acid); a polyetherester; a polyacetal; a polycyanoacrylate; a poly(oxyethylene)/poly(oxypropylene) copolymer, polysuccinimde, chitosan, lignosulfonates; chitins; a polysaccharide, a polyphosphazene, a protein, a lipid, or combinations thereof. In an embodiment, such a combination may take the form of a co-polymer and/or a physical blend. In an embodiment a polymer suitable for use in the present disclosure is a natural polymer such as protein-based polymers and polysaccharides. Examples of such polymers include collagen, albumin, gelatin, agarose, alginate, carrageenan, hyaluronic acid, dextran, chitosan and cyclodextrins. In an embodiment the polymer is a polyanhydride such as poly(sebaci cid), poly(adipic acid), and poly(terphtalic) acid. In an embodiment, the polymer is a polyamide such as a poly(imino carbonate) or a polyaminio acid. In an embodiment, the polymer is phosphorus based such as a polyphosphate, a polyphosphonate or a polyphosphazene. In an embodiment, the polymer comprises an α-prolamin.

In an embodiment, the degradable polymer comprises polysaccharides, such as starches, cellulose, dextran, substituted or unsubstituted galactomannans, guar gums, high-molecular weight polysaccharides composed of mannose and galactose sugars, heteropolysaccharides obtained by the fermentation of starch-derived sugar (e.g., xanthan gum), diutan, scleroglucan, derivatives thereof, or combinations thereof.

In an embodiment, the polymer comprises guar or a guar derivative. Examples of guar derivatives suitable for use in the present disclosure include without limitation hydroxypropyl guar, carboxymethylhydroxypropyl guar, carboxymethyl guar, hydrophobically modified guars, guar-containing compounds, or combinations thereof.

In an embodiment, the polymer comprises cellulose or a cellulose derivative. Examples of cellulose derivatives suitable for use in the present disclosure include without limitation cellulose ethers, ethyl cellulose, cellulose acetate, cellulose acetate propionate carboxycelluloses, carboxyalkylhydroxyethyl celluloses, hydroxyethylcellulose, hydroxypropylcellulose, carboxymethylhydroxyethylcellulose, carboxymethylcellulose, or combinations thereof.

In an embodiment, the polymer comprises a starch. Examples of starches suitable for use in the present disclosure include without limitation native starches, reclaimed starches, waxy starches, modified starches, pre-gelatinized starches, or combinations thereof.

Biodegradable shape memory polymers, such as those commercialized by nmemoScience in Aachen, Germany, or those described in U.S. Pat. No. 5,189,110 or U.S. Pat. No. 5,139,832, each of which is disclosed herein in its entirety, may also be employed.

In an embodiment, a degradable polymer as may be suitable for use in making the components and/or materials which may form the BSS/BAIESS include may be made by any suitable process and/or combination of processes. For example, in an embodiment such a degradable polymer may be made or otherwise formed in a process comprising melt polymerization. For example, in an embodiment where the polymer comprises one or more monomer having a "ring" structure (e.g., a poly(lactide), a poly(glycolide), a poly(lactide-co-glycolide), a poly(lactic acid), a poly(glycolic acid), and/or a poly(lactid acid-co-glycolic acid)), melt polymerization may result in the opening of such ringed structure. In an embodiment, one or more of the degradable polymers, for example, as disclosed herein, may be available with, alternatively, without carboxylic acid end groups. In an embodiment where the end group of the degradable polymer is not a carboxylic acid (for example, where the end group comprises an ester), the resultant polymer may be referred to herein as a blocked or capped polymer. In an embodiment where the degradable polymer has a terminal carboxylic group, the resultant polymer may be referred to as an unblocked or uncapped polymer. In embodiments, the degradable polymers may be characterized as linear polymers, star polymers, or combinations thereof. Additionally, in an embodiment, the degradable polymers may be characterized as high molecular weight polymers. For example, not intending to be bound by theory, such high molecular weight polymers may improve the strength of the platform and/or extend the time duration association with degradation and/or biodegradation (e.g., bioabsorption or resorption time). In an alternative embodiment, low molecular weight polymers may be utilized where degradation and/or biodegradation time is preferably less and/or where less strength is necessary. As will be appreciated by those of skill in the art, lactide monomers and/or the lactide portion of a given polymer comprise an asymmetric carbon. Suitable, racemic DL-, L-, and D-polymers, for example, as may be suitable for inclusion in the composition, are commercially available. Not intending to be bound by theory, the L-polymers may be relatively more crystalline and, as such, may degrade and/or biodegrade (e.g., adsorb, absorb, resorb, and/or dissipate) more slowly than DL-polymers. Copolymers comprising glycolide and DL-lactide or L-lactide as well as copolymers of L-lactide and DL-lactide are also commercially available. Additionally, homopolymers of lactide or glycolide are commercially available. Star polymers of lactide or glycolide or lactide/glycolide copolymers are also commercially available.

In an embodiment where the degradable polymer comprises a poly(lactide-co-glycolide), a poly(lactide), or a poly(glycolide), the lactide and/or the glycolide may be present within the polymer may in a suitable amount. In an embodiment, the degradable polymer contains a lactide in an amount of from about 0 to about 100 mole %, alternatively, from about 40 to about 100 mole %, alternatively, from about 50 to about 100 mole %, alternatively, from about 60 to about 100 mole %, alternatively, from about 70 to about 100 mole %, alternatively, alternatively, from about 80 to about 100 mole %, and/or glycolide in an amount of from about 0 to about 100 mole %, alternatively, from about 0 to about 60 mole %, alternatively, from about 10 to about 40 mole %, alternatively, from about 20 to about 40 mole %, alternatively, from about 30 to about 40 mole %. In an embodiment, the degradable polymer contains lactide and glycolide in an amount summing 100 mole %, alternatively, about 99%, alternatively, about 95%, alternatively, about 90%. In an embodiment where the degradable polymer comprises lactide and glycolide, the lactide and glycolide may be present in a ratio of about 85:15 poly(lactide-co-glycolide), alternatively, about 75:25 poly(lactide-co-glycolide), alternatively, about 65:35 poly(lactide-co-glycolide), alternatively, about 50:50 poly(lactide-co-glycolide), where the ratios are mole ratios.

In an embodiment where the degradable polymer comprises a poly(lactide-co-glycolide), a poly(lactide), or a poly(glycolide), the polymer may be characterized as having an intrinsic viscosity of from about 0.15 to about 1.5 dL/g, alternatively, from about 0.25 to about 1.5 dL/g, alternatively, from about 0.25 to about 1.0 dL/g, alternatively, from about 0.25 to about 0.8 dL/g, alternatively, from about 0.25 to about 0.6 dL/g, alternatively, from about 0.25 to about 0.4 dL/g, for example, as measured in chloroform at a concentration of 0.5 g/dL at 30° C.

In an additional and/or alternative embodiment, the polymer may comprise a nondegradable polymer. Examples of a suitable nonbiodegradable polymer include, but are not limited to, poly(ethylene vinyl acetate), poly(vinyl acetate), silicone polymers, polyurethanes, polysaccharides such as a cellulosic polymers and cellulose derivatives, acyl substituted cellulose acetates and derivatives thereof, copolymers of poly(ethylene glycol) and poly(butylene terephthalate), polystyrenes, polyvinyl chloride, polyvinyl fluoride, poly(vinyl imidazole), chorosulphonated polyolefins, polyethylene oxide, copolymers thereof, or combinations thereof. For example, in an embodiment as will be disclosed herein the platform or a portion or component thereof may comprise both degradable and non-degradable portions.

In an embodiment, the polymer (e.g., a degradable or nondegradable polymer) may be characterized as mucoadhesive or bioadhesive. As used herein, the terms mucoadhesive and/or bioadhesive may refer to materials tending to exhibit adhesion to biological tissue, such as mucosae (e.g., one or more mucous membranes). In some instances, the platform, one or more of the component and/or materials utilized to form the platform, and/or the BSS/BAIESS may be coated with a mucoadhesive, which may or may not be a polymer. Additionally, in an embodiment the polymer may be characterized as charged and/or ionic in character, for example, may alter the interaction of the polymer(s) with one or more bodily surfaces.

In another additional or alternative embodiment, the polymer may comprise a natural polymer. Suitable natural polymers include, but are not limited to, proteins (such as zein, modified zein, casein, chitin, gelatin, gluten, serum albumin, and/or collagen), polysaccharides (such as cellulose, dextrans, and/or polyhyaluronic acid), or combinations thereof. Additionally, in an embodiment such polysaccharides may be present in the form of a hydrogel or sol-gel mixtures.

In an embodiment, the composition utilized to form the platform may further comprise one or more suitable additives. Additionally or alternatively, in an embodiment the platform one or more components and/or materials utilized to form the platform may be treated with one or more suitable additives (e.g., the additives may be applied there to). Examples of suitable additives as may be included within the composition, added to the composition, and/or otherwise applied to the platform, include, but are not limited to, preservatives, buffers, binders, disintegrants, lubricants, plasticizers, solvents, sterilizing agents, primers, and any other excipients, for example, as may be necessary and/or advantageous to impart a desired structural, functional and/or processing characteristic to the components and/or materials utilized to form the platform, the platform, and/or the BSS/BAIESS. For example, in an embodiment a plasticizer and/or solvent may be included in the composition, for example, for the purpose of softening (e.g., improving malleability and or conformability of) the polymer. Examples of a suitable plasticizer and/or solvent include, but are not limited to acetone, methyl ethyl ketone, ethyl lactate, ethyl acetate, dichloromethane, ethyl acetate/alcohol blends, or combinations thereof. In such an embodiment, the plasticizer or solvent may diffuse or otherwise be released from the platform after deployment, for example, allowing the platform (e.g., which has conformed to the shape of the lumen, cavity, orifice, or the like into which the BSS/BAIESS has been deployed) to harden such that the BSS/BAIESS substantially conforms to the shape of the environment into which it has been deployed.

In an additional embodiment, the additive may comprise a mucoadhesive. Not intending to be bound by theory, such a mucoadhesive may enhance contact between the platform and/or the BSS/BAIESS and one or more tissues at the site at which the BSS/BAIESS is deployed. In an embodiment, the mucoadhesive may comprise a polymer. Examples of mucoadhesive polymers include, but are not limited to, homopolymers of acrylic acid monomers such as polyacrylic acid and any of its pharmaceutically acceptable salts; copolymers of acrylic acid and methacrylic acid, styrene, or vinyl ethers; vinyl polymers such as polyhydroxyethyl acrylate, polyhydroxyethyl methacrylate, polyvinyl alcohol, and polyvinyl pyrrolidone; cellulosic derivatives such as methyl cellulose, ethyl cellulose, hydroxyethyl cellulose, hydroxypropyl cellulose, hydroxypropylmethyl cellulose, and carboxymethyl cellulose; polysaccharides such as alginic acid, sodium alginate, and tragacanth gum; collagen; gelatin; or combinations thereof.

In an embodiment, the AI may be incorporated with the platform (e.g., with and/or within the polymer composition utilized to form the platform), as will be discussed herein.

In an embodiment, the platform may comprise any suitable form and/or configuration, for example, as may be dependent upon the intended use of the BSS/BAIESS. In an embodiment, the platform may be characterized as exhibiting a desired degree of flexibility, conformability, and/or malleability, alternatively, the platform may be characterized as exhibiting a desired degree of rigidity. In an embodiment, the platform may be characterized as mechanically or structurally active. For example, in an embodiment, at least a portion of a platform may be configured such that the platform will apply a radially outward force (e.g., a biasing force), such as in an embodiment where the BSS/BAIESS is deployed within a lumen, as will be disclosed herein. Alternatively, in an embodiment, at least a portion of a platform may be configured such that the platform will apply a radially inward force (e.g., a biasing force), such as in an embodiment where the BSS/BAIESS is deployed around (e.g., at least partially encompassing) a tissue, as will be disclosed herein.

In an embodiment, the platform may be radially expandable and/or contractible with respect to a longitudinal axis and a central passage or through bore (e.g., formed by a cylinder wall). For example, in an embodiment, the platform may be configured so as to expand radially outward and/or to exhibit a radially outward force or bias (e.g., a radially outward mechanical activity) with respect to the longitudinal axis when uninhibited from such expansion, as will be disclosed herein. Alternatively, in an embodiment the platform may be configured to contract radially inward and/or the exhibit a radially inward force or bias (e.g., a radially inward mechanical activity) with respect to the longitudinal axis when uninhibited from such contraction, as will also be disclosed herein.

In an embodiment the platform comprises a hollow, generally cylindrical structure having a longitudinal axis. In the embodiment of Figure 1, such a hollow, cylindrical platform may comprise a relatively constant diameter with respect to the longitudinal axis. Alternatively, a hollow, generally cylindrical platform may comprise a variable diameter. For example, a generally cylindrical platform may be tapered at one or both longitudinal ends, flared at one or both ends, at least partially conical (e.g., having a diameter that changes over the longitude of the platform), or combinations thereof. As will be disclosed herein, the size, shape, mechanical activity, strength characteristics, and other characteristics associated with the platform may vary depending upon various considerations including, but not limited to, the intended site/locus of deployment for the platform, the intended purpose/use for which the platform is deployed, the intended mode of deployment, various related considerations, or combinations thereof.

Referring to Figures 1 through 5, various platform configurations are generally illustrated. As will be disclosed herein, one or more of the general configurations disclosed with reference to Figures 1 through 5 may be modified, for example, for a particular purpose or use. For example, in the embodiments of Figures 1 through 5, the platform generally comprises cylinder. In the embodiment of the Figure 1, the generally cylindrical platform is formed from a mesh-like material formed by a plurality of strands 105, for example as described herein. In alternative embodiments, a platform like the platform of Figure 1 may be formed from any other suitable mesh-like material, for example, having a suitable design.

In the embodiment of Figure 2, the generally cylindrical platform is formed from a plurality of struts joined at terminus or ends thereof. In various embodiments, such a cylindrical platform may be formed from any suitable number of similar struts, for example, being joined at the ends thereof, alternatively, being joined at a point between the ends thereof.

In the embodiment of Figure 3, the generally cylindrical platform is formed from helical/spiral strand. In the embodiment of Figure 3, the platform comprises a single, such strand. In an additional or alternative embodiment, a platform may be formed from a plurality of helical and/or spiral strands. For example, such a plurality of helical/spiral strands may coil in the same direction (e.g., a double-helix) or in opposing directions.

In the embodiment of Figure 4, the generally cylindrical platform is formed from a sheet, a film, or a membrane. In an embodiment, the platform may be formed from a sheet, film, or membrane that has been folded and/or wrapped so as to yield the generally cylindrical configuration; alternatively, the sheet, film, or membrane may be formed having such a generally cylindrical (e.g., tubular) structure.

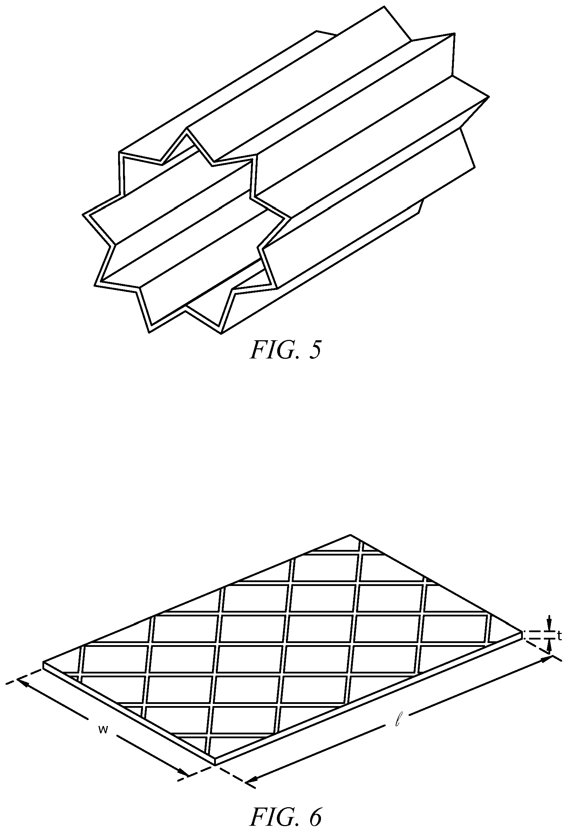

In an additional embodiment, for example, in the embodiment of Figure 5, such a generally cylindrical platform may comprise one or more pleats. In such an embodiment, the number and/or size of such pleats may be varied, for example, dependent upon the intended use of the BSS/BAIESS, the configuration of the platform, processing parameters associated with making and/or use the platform and/or the BSS/BAIESS, or combinations thereof, as will be disclosed herein

In an embodiment, where the platform comprises such a hollow, cylindrical platform, the platform may be characterized as having a variable cross-sectional conformation, for example, the radial cross-section of the platform may vary such that the platform exhibits mechanical activity (for example, as capable of expansion and/or contraction for placement at a site of deployment, for variability in placement, for the application of a force to the surrounding tissue, or the like). For example, in an embodiment such a platform may be radially expandable, for example, the platform may be configured so as to expand radially outward when not retained in a relatively unexpanded conformation. In such an embodiment, the platform may be configured to apply a radially-outward force (for example, to a lumen, passageway, or other opening) upon deployment, as will be disclosed herein. In an embodiment, the radially outward force applied by the platform may be varied, for example, dependent upon the intended use of the BSS/BAIESS, the site of deployment, or other factors as may be appreciated by one of skill in the art upon viewing this disclosure. Additionally, in an embodiment the radially outward force applied by the platform may be variable over time, for example, as may result from the degradation of a portion of the platform. For example, in an embodiment, the radially outward force applied by the platform may increase, alternatively, decrease, over time. In an embodiment, a nonbiodegradable component (alternatively, a component being relatively slowly degradable) may form an outwardly-biased cylinder which is held in a partially contracted state by one or more biodegradable bands, for example, such that as such bands degrade, the nonbiodegradable cylinder may expand (e.g., over time). For example, such a nonbiodegradable cylinder (alternatively, a cylinder being relatively slowly degradable) may be configured to expand (e.g., to increase in expansion) as a tissue heals and/or as swelling subsides.

In an alternative embodiment, where the platform comprises such a hollow, cylindrical platform, the platform may be characterized as having a variable cross-sectional conformation (for example, as capable of expansion and/or contraction for placement at a site of deployment, for variability in placement, for the application of a force, or the like). Additionally, in an embodiment the platform, or portions or components thereof may comprise a "locking" profile," such as a ratchet, teeth, grooves, or the like, configured to lock in an expanded or contracted state upon deployment. For example, in an embodiment such a platform may be radially contractible, for example, the platform may be configured so as to expand radially inward when not retained in a relatively expanded conformation. In such an embodiment, the platform may be configured to apply a radially-inward force (for example, to occlude or compress or narrow a lumen, passageway, or other opening) upon deployment, as will be disclosed herein. In an embodiment, the radially inward force applied by the platform may be varied, for example, dependent upon the intended use of the BSS/BAIESS, the site of deployment, or other factors as may be appreciated by one of skill in the art upon viewing this disclosure. Additionally, in an embodiment the radially inward force applied by the platform may be variable over time, for example, as may result from the degradation of a portion of the platform. For example, in an embodiment, the radially inward force applied by the platform may increase, alternatively, decrease, over time. In an embodiment, a nonbiodegradable component (alternatively, a component being relatively slowly degradable) may form an inwardly-biased cylinder which is held in a partially expanded state by one or more biodegradable bands, for example, such that as such bands degrade, the nonbiodegradable cylinder may contract (e.g., over time). For example, such a nonbiodegradable cylinder (alternatively, a cylinder being relatively slowly degradable) may be configured to contract (e.g., to narrow) over time.

In an embodiment where the platform comprises such a hollow, cylindrical platform, the platform may be suitably sized, for example, as may be dependent upon the intended use of the BSS/BAIESS. In an embodiment, the platform may be adaptable to varying cross-sectional passageways. In an embodiment, the platform may comprise a suitable diameter and a suitable length. For example, in an embodiment the outer diameter (*d*) may be in the range of from about 5 mm to about 3 cm, alternatively, from about 5 mm to about 1 cm, alternatively, from about 5 mm to about 20 mm. Also, in an embodiment the length (*l*) may be in a range of from about 50 mm to about 5 cm, alternatively, from about 1 cm to about 4 cm, alternatively, from about 2 cm to about 3 cm.

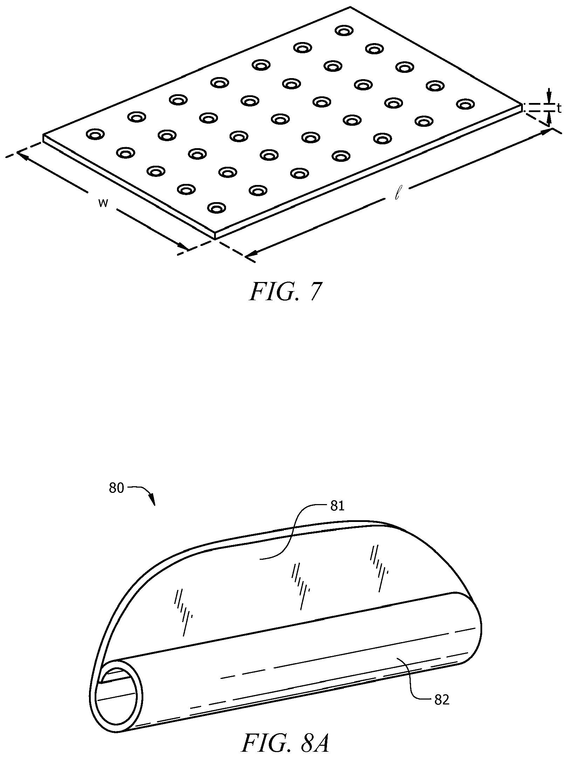

Alternatively, in an embodiment the platform generally comprises a sheet. In an embodiment, the platform generally comprises one or more thicknesses of a suitable sheet-forming material, for example such as a mesh-like material, a film-like material, a membrane material, as disclosed herein, or combinations thereof. For example, in the embodiment of Figure 6, such a sheet may comprise one or more thicknesses of a mesh-like material, for example, as disclosed herein. Alternatively, in the embodiment of Figure 7, the sheet may comprise one or more thicknesses of a film-like or membrane-like material.

In an embodiment where the platform comprises such a sheet, the sheet may be employed to form one or more suitable structures. For example, such a sheet may be rolled, folded, bended, the like, or combinations thereof. In an embodiment, a platform comprising a sheet may be characterized as exhibiting a desired degree of conformability. For example, the sheet may be configured to be rolled, crimped, folded, stretched, compressed, pleated, or combinations thereof. Additionally or alternatively, in an embodiment the sheet may be configured so as to exhibit a tendency to remain in a first conformation when placed in such first conformation. For example, the sheet may be characterized as malleable. Alternatively, the sheet may be configured so as to exhibit a tendency to return to a first conformation when placed in a second conformation.

For example, in an embodiment, a sheet may be configured to be rolled. For example, in an embodiment, when such a sheet is rolled, the sheet may be configured so as to exhibit a tendency to unroll, for example, so as to exhibit a radially outward force. As such, such a platform may be characterized as radially expandable, for example, the platform may be configured so as to expand radially outward (e.g., unroll) when not retained in a rolled conformation. In an embodiment, retaining members (e.g., straps or bands) may be biodegradable such that unrolling may occur in a controlled and/or delayed fashion as directed by a given scenario. In such an embodiment, the platform may be configured to apply a radially-outward force (for example, to a lumen, passageway, or other opening) upon deployment, as will be disclosed herein.

In an alternative embodiment, a platform may be configured such that the sheet exhibits a tendency to roll, for example, so as to exhibit a radially inward force (e.g., a centripetal force). As such, the sheet may be characterized as radially contractible, for example, so as to contract radially inward (e.g., roll) when not retained in an unrolled and/or partially unrolled conformation. In an embodiment, biodegradable retaining members may prevent roll-up, such that rolling may occur in a controlled and/or delayed fashion as directed by a given scenario. In such an embodiment, the sheet may be configured to apply a radially-inward force (for example, so as to constrict a lumen, passageway, or other opening) upon deployment, as will be disclosed herein.

In another embodiment, a sheet may be configured to be stretched. For example, in an embodiment, when such a sheet is stretched, the sheet may exhibit a desired tensile strength and/or a desired bias, for example, such that the sheet exhibits a tendency to shrink in at least dimension (e.g., an elastomeric or spring-like behavior).

In still another embodiment, a sheet may be configured to be crimped or folded. For example, in an embodiment a sheet may be configured such that when the sheet is placed (e.g., folded, crimped, squeezed, or the like), the sheet exhibits a tendency to remain in that configuration. As such, in an embodiment a sheet may be formed into a suitable shape with the expectation that the sheet will remain in that shape. In an embodiment, a sheet comprises a mating and/or interfacing component, for example, that forms a lock (e.g., a zip-tie-like structure).

Additionally, in an embodiment, a sheet may be configured so as to be formed into a particular shape. For example, in various embodiments, a sheet may be formed into a suitable shape (e.g., rolled) and, two or more portions of such a sheet bonded together so as to remain in the desired shaped. For example, a sheet may be bonded via the application of a suitable adhesive, by sewing (e.g., utilizing a surgical thread or suture, such as a bioabsorbable suture), by annealing (e.g., by heat and/or pressure), or by combinations thereof.

In an embodiment, a platform comprising a sheet may comprise any suitable shape. Examples of suitable shapes include, but are not limited to a square, a rectangle, an oval, a circle, a parallelogram, a triangle, a rhombus, a pentagon, or the like. In an embodiment, a platform may be suitably sized. For example, in the embodiment of Figure 7, the sheet is generally rectangular in shape and may have a length (/) in the range of from about 50 mm to about 5 cm, alternatively, from about 1 cm to about 4 cm, alternatively, from about 2 cm to about 3 cm. Also, in an embodiment the width (w) may be in a range of from about 50 mm to about 5 cm, alternatively, from about 1 cm to about 4 cm, alternatively, from about 2 cm to about 3 cm. Also, in an embodiment the sheet may have a thickness (t) in the range of from about 1 mm to about 5 mm, alternatively, from about 2 mm to about 4 mm, alternatively, from about 2 mm to about 3 mm.

Additionally, in an embodiment the sheet may be sizable. For example, the sheet may be produced in varying sizes (e.g., small, medium, or large), for example, as may be suitable dependent upon the intended use of the BSS/BAIESS. Additionally or alternatively, in an embodiment the sheet may be trimmable so as to achieve a desired size. For example, such a sheet may be trimmed, cut, shaped, or other altered so as to yield a desired size and/or configuration, for example, utilizing conventional and readily-available tools such as scissors, knives, scalpels, or the like.

In an embodiment, the platform may further comprise one or more additional components. For example, such additional components may be configured to anchor the platform or a portion thereof, to retain the platform in a given orientation at and/or within the site of deployment (e.g., locks, bands, straps, or ties), to retain the platform in a given conformation, or the like. In an embodiment, such an additional component may be characterized as a filament, a flange-like component, a collar-like component, an extension, or the like. In an embodiment, such one or more additional components may extend through the platform, radially inward within the platform, radially outward from a portion of the platform, tangentially from the platform, or combinations thereof. In an embodiment, the platform may comprise a plurality of operably joined components. Alternatively, the platform may be formed as a unitary structure.

In another alternative embodiment, the platform may comprise a filler material. In such an embodiment the filler material may comprise a plurality of particles (e.g., a particulate material). In an embodiment, the particles may have a size ranging from about 1 µm to about 2,500 µm, alternatively, from about 10 µm to about 500 µm, alternatively, from about 25 µm to about 100 µm, alternatively, from about 50 µm to about 75 µm.

In an embodiment, the particulates may be suspended (e.g., for delivery or deployment) in a carrier fluid, such as a gel, a saline solution, an aqueous solution, or combinations thereof.

In an embodiment, the AI may comprise any suitable pharmaceutically active component, any suitable active pharmaceutical ingredient (API), any suitable therapeutically-active component, any-suitable prophylactically-active component, any suitable cosmetic component, any suitable material that is safe for human use and has biological activity, or combinations thereof. The AI(s) may be adherent to the platform itself, or incorporated or otherwise surrounded, encompassed, or sequestered within and/or by the platform. Not intending to be bound by theory, AI(s) may be incorporated into the BAIESS along with co-ingredients to alter, delay, hasten, or otherwise manipulate or control the rate of release and activity within the host tissue.

While one or more of the embodiments disclosed herein may refer to a BAIESS as comprising one or more AIs, it is also contemplated that one or more AIs may be added to a BSS (where an AI is generally not present), for example, effectively rendering the BSS as a BAIESS as referenced herein. Additionally, in such an embodiment, an AI may be applied to a BSS prior to deployment, substantially contemporaneously with deployment (e.g., during the deployment procedure), following deployment, or combinations thereof. In such an embodiment, a BSS may be configured to uptake one or more AIs. For example, in such an embodiment, the BSS may comprise pores, bonding agents (e.g., adhesives, molecular binders, or otherwise), for example, thereby enabling an AI brought into contact with the BSS (e.g., with the platform) to be retained thereon.

In an embodiment, the active ingredient may comprise any ingredient suitable for the treatment, prevention, rehabilitation, therapy, alteration, minimization, amelioration, camouflage, or the like of a condition in a subject (e.g., a human "patient"), examples of which include but are not limited to chronic sinusitis, obstructive sleep apnea, postoperative inflammation/scarring/non-healing wounds, stenosis of a tubular structure due to injury or burn, middle ear inflammatory disease, Eustachian tube dysfunction, nasolacrimal duct obstruction, pharyngeal stenosis, spinal stenosis, spinal rootlet compression, esophageal stenosis, incompetence of the lower esophageal sphincter, laryngeal stenosis, tracheal stenosis, reactive airway disease, biliary tract stenosis, pancreatic duct stenosis, ureteral stenosis, urethral stricture, Fallopian tube obstruction, contraception, sexually-transmitted diseases in the male and the female, interruption of the Fallopian tubes or the vas deferens due to prior contraceptive surgical procedures, vascular occlusion or stenosis, malignancy requiring chemotherapy, physical defects requiring skin grafting or microvascular free flap tissue transfer, temporary or permanent change in eye color, and in rejuvenation of the aging face.

Examples of an active ingredient an may be included within the BAIESS include, but are not limited to, anticholinergic agents, anti-infective agents (e.g., an antibiotic, such as antibacterial agents, antifungal agents, antiparasitic agents, antiviral agents, antiseptics or combinations thereof), anti-inflammatory agents (such as steroidal and/or nonsteroidal anti-inflammatory agents), antiscarring or antiproliferative agents, chemotherapeutic/antineoplastic agents, cytokines, decongestants, extracellular signaling/intracellular signaling molecules, healing-promotion agents and vitamins, hemostatic agents, hormones, hyperosmolar agents, immunoglobulins, immunomodulators, immunosuppressive agents, leukotriene modifiers, mast cell stabilizers, mitotic inhibitors, mucolytics, muscle relaxants, narcotic analgesics, non-narcotic analgesics, nucleic acids, other peptides, other proteins including potential allergens for immunotherapy (e.g., pollen), proton-pump inhibitors, sclerosing agents, tyrosine kinase inhibitors, vasoactive agents or combinations thereof. Additionally, anti-sense nucleic acid oligomers or other direct transactivation and/or transrepression modifiers of mRNA expression, transcription, and protein production may also be used.

In an embodiment, suitable anticholinergic agents may generally include antimuscarinic agents, ganglionic blockers, neuromuscular blockers, or combinations thereof. Examples of anticholinergic agents include, but are not limited to, ipratroprium bromide, dicycloverine, atropine, benztropine, ipratropium, oxitropium, tiotropium, glycopyrrolate, oxy butnin, tolterodine, diphenhydramine, and dimenhydrinate.

In an embodiment, suitable antihistamines may generally include H₁-receptor antagonists, H₂-receptor antagonists, H₃-receptor antagonists, H₄-receptor antagonists, histidine decarboxylase inhibitors, mast cell stabilizers, or combinations thereof. Examples of suitable H₁-receptor antagonists include, but are not limited to, azelastine, diphenhydramine, chlorpheniramine, meclozine, promethazine, loratadine, desloratadine, fexofenadine, cetirizine, levocetirizine, olopatadine, brompheniramine, buclizine, bromodiphenhydramine, carbinoxamine, chlorpromazine, cyclizine, chlorpheniramine, clemastine, cyproheptadine, dexbromheniramine, deschlorpheniramine, dexchlorpheniramine, dimenhydrinate, dimetindene, doxylamine, ebastine, embramine, orphenadrine, phenindamine, pheniramine, phenyltoloxamine, pyrilamine, quetiapine, rupatadine, tripelennamine, and trirolidine. Examples of suitable H₂-receptor antagonists include, but are not limited to, cimetidine, famotidine, lafutidine, nizatidine, ranitidine, and roxatidine. Examples of suitable H₃-receptor antagonists include, but are not limited to, ciproxifan, clobenpropit, conessine, and thioperamide. Examples of suitable H₄-receptor antagonists include, but are not limited to, thioperamide. Examples of suitable histidine decarboxylase inhibitors include tritiqualine and catechin. Examples of suitable mast cell stabilizers include cromoglicate, medocromil, cromolyn sodium, and β2 adrenergic agonists.

Examples of suitable antibacterial agents include, but are not limited to, aminoglycosides, amphenicols, ansamycins, β-lactams, lincosamides, macrolides, nitrofurans, quinolones (e.g., levofloxacin), sulfonamides, sulfones, tetracyclines, vancomycin, and any of their derivatives, or combinations thereof.

In an embodiment, suitable β-lactams include, but are not limited to, carbacephems, carbapenems, cephalosporins, cephamycins, monobactams, oxacephems, penicillins, and any of their derivatives.

In an embodiment, suitable penicillins include, but are not limited to, amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine, penicillin G potassium, penicillin G procaine, penicillin N, penicillin O, penicillin V, penicillin V benzathine, penicillin V hydrabamine, penimepicycline, phenethicillin potassium, piperacillin, pivampicillin, propicillin, quinacillin, sulbenicillin, sultamicillin, talampicillin, temocillin, and ticarcillin. In an embodiment, penicillins may be combined with clavulanic acid. An example of a suitable combination of a penicillin and clavulanic acid is Augmentin™ (amoxicillin and clavulanic acid).

Examples of suitable antifungal agents include, but are not limited to, allylamines, imidazoles, polyenes, thiocarbamates, triazoles, and any suitable derivatives thereof.

Examples of suitable antiparasitic agents include atovaquone, clindamycin, dapsone, iodoquinol, metronidazole, pentamidine, primaquine, pyrimethamine, sulfadiazine, trimethoprim/sulfamethoxazole, trimetrexate, and combinations thereof.

Examples of suitable antiviral agents include, but are not limited to, acyclovir, famciclovir, valacyclovir, edoxudine, ganciclovir, foscamet, cidovir (vistide), vitrasert, formivirsen, HPMPA (9-(3-hydroxy-2-phosphonomethoxypropyl)adenine), PMEA (9-(2-phosphonomethoxyethyl)adenine), HPMPG (9-(3-Hydroxy-2-(Phosphonomet-hoxy)propyl)guanine), PMEG (9-[2-(phosphonomethoxy)ethyl]guanine), HPMPC (1 -(2-phosphonomethoxy-3-hydroxypropyl)-cytosine), ribavirin, EICAR (5-ethynyl-1-beta-D-ribofuranosylimidazole-4-carboxamine), pyrazofurin (3-[beta-D-ribofuranosyl]-4-hydroxypyrazole-5-carboxamine), 3-Deazaguanine, GR-92938X (1-beta-D-ribofuranosylpyrazole-3,4-dicarboxami-de), LY253963 (1,3,4-thiadiazol-2-yl-cyanamide), RD3-0028 (1,4-dihydro-2,3-Benzodithiin), CL387626 (4,4'-bis[4,6-d]3-aminophenyl-N-,N-bis(2-carbamoylethyl)-sulfonilimino]-1-,3,5-triazin-2-ylamino-biphenyl-2-,2'-disulfonic acid disodium salt), BABIM (Bis[5-Amidino-2-benzimidazoly-1]-methane), NIH351, and combinations thereof.

In an embodiment, an anti-inflammatory agent may comprise a steroidal anti-inflammatory agent (e.g., a corticosteroid). Examples of suitable steroidal anti-inflammatory agents include 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, any of their derivatives, and combinations thereof.

In an additional or alternative embodiment, the anti-inflammatory agent may comprise a nonsteroidal anti-inflammatory agent. Examples of suitable nonsteroidal anti-inflammatory agents include, but are not limited to, COX inhibitors (COX-1 or COX nonspecific inhibitors) (e.g., salicylic acid derivatives, aspirin, sodium salicylate, choline magnesium trisalicylate, salsalate, diflunisal, sulfasalazine and olsalazine; para-aminophenol derivatives such as acetaminophen; indole and indene acetic acids such as indomethacin and sulindac; heteroaryl acetic acids such as tolmetin, dicofenac and ketorolac; arylpropionic acids such as ibuprofen, naproxen, flurbiprofen, ketoprofen, fenoprofen and oxaprozin; anthranilic acids (fenamates) such as mefenamic acid and meloxicam; enolic acids such as the oxicams (piroxicam, meloxicam) and alkanones such as nabumetone); selective COX-2 inhibitors (e.g., diaryl-substituted furanones such as rofecoxib; diaryl-substituted pyrazoles such as celecoxib; indole acetic acids such as etodolac and sulfonanilides such as nimesulide); or combinations thereof.

Examples of anti-scarring agents include, but are not limited to, silicon and vitamin E and derivatives thereof.

Examples of antiseptics include, but are not limited to chlorhexadine, benzalkonium chloride, octenidine, boric acid, cetyl trimethylammonium bromide, cetylpyridium chloride, benzethonium chloride, povidone, betadine, polyhexanide, iodine and derivatives thereof, and combinations thereof.

Examples of suitable chemotherapeutic/antineoplastic agents include, but are not limited to antitumor agents (e.g., cancer chemotherapeutic agents, biological response modifiers, vascularization inhibitors, hormone receptor blockers, cryotherapeutic agents or other agents that destroy or inhibit neoplasia or tumorigenesis) such as alkylating agents or other agents which directly kill cancer cells by attacking their DNA (e.g., cyclophosphamide, isophosphamide), nitrosoureas or other agents which kill cancer cells by inhibiting changes necessary for cellular DNA repair (e.g., carmustine (BCNU) and lomustine (CCNU)), antimetabolites and other agents that block cancer cell growth by interfering with certain cell functions, usually DNA synthesis (e.g., 6 mercaptopurine and 5-fluorouracil (5FU), antitumor antibiotics and other compounds that act by binding or intercalating DNA and preventing RNA synthesis (e.g., doxorubicin, daunorubicin, epirubicin, idarubicin, mitomycin-C and bleomycin) plant (vinca) alkaloids and other anti-tumor agents derived from plants (e.g., vincristine and vinblastine), steroid hormones, hormone inhibitors, hormone receptor antagonists and other agents which affect the growth of hormone-responsive cancers (e.g., tamoxifen, herceptin, aromatase ingibitors such as aminoglutethamide and formestane, trriazole inhibitors such as letrozole and anastrazole, steroidal inhibitors such as exemestane), antiangiogenic proteins, small molecules, gene therapies and/or other agents that inhibit angiogenesis or vascularization of tumors (e.g., meth-1, meth-2, thalidomide), bevacizumab (Avastin), squalamine, endostatin, angiostatin, Angiozyme, AE-941 (Neovastat), CC-5013 (Revimid), medi-522 (Vitaxin), 2-methoxyestradiol (2ME2, Panzem), carboxyamidotriazole (CAI), combretastatin A4 prodrug (CA4P), SU6668, SU11248, BMS-275291, COL-3, EMD 121974, IMC-1C11, IM862, TNP-470, celecoxib (Celebrex), rofecoxib (Vioxx), interferon alpha, interleukin-12 (IL-12) or any of the compounds identified in Science Vol. 289, Pages 1197-1201 (Aug. 17, 2000), which is incorporated herein by reference in its entirety, biological response modifiers (e.g., interferon, bacillus calmette-guerin (BCG), monoclonal antibodies, interluken 2, granulocyte colony stimulating factor (GCSF), etc.), PGDF receptor antagonists, herceptin, asparaginase, busulphan, carboplatin, cisplatin, carmustine, cchlorambucil, cytarabine, dacarbazine, etoposide, flucarbazine, flurouracil, gemcitabine, hydroxyurea, ifosphamide, irinotecan, lomustine, melphalan, mercaptopurine, methotrexate, thioguanine, thiotepa, tomudex, topotecan, treosulfan, vinblastine, vincristine, mitoazitrone, oxaliplatin, procarbazine, streptocin, taxol or paclitaxel, taxotere, analogs/congeners, derivatives of such compounds, and combinations thereof.

Examples of cytokines include, but are not limited to interferon (such as, but not limited to type I, type II, and type III interferons) and interleukins.

Examples of proton-pump inhibitors include, but are not limited, dexlansoprazole, esomeprazole, lansoprazole, omeprazole, pantoprazole, rabeprazole, or combinations thereof.

Examples of decongestants include, but are not limited to, epinephrine, pseudoephedrine, oxymetazoline, phenylephrine, tetrahydrozolidine, xylometazoline, or combinations thereof.

Examples of healing-promotion agents and vitamins include, but are not limited to, retinoic acid, vitamin A, vitamin D, vitamin E, vitamin K, and derivatives thereof.

Examples of hemostatic agents include, but are not limited to, aminocaproic acid and desmopressin.

Examples of potential hormones include, but are not limited to, androgens (such as aldosterone, testosterone, or dehydroepiandrosterone), anti-androgens, estrogens, anti-estrogens, progesterone, estradiol, GnRH analogs, steroids, sterols, growth hormones, anti-diuretic hormone, melatonin, serotonin, thyroxine, triiodothyronine, calcitonin, thyroid-stimulating hormone (TSH), parathyroid hormone, glucagon, epinephrine, norepinephrine, dopamine, oxytocin, insulin, insulin-like growth factor, and analogs thereof.

In an embodiment where it is desirable to remove water from a tissue (e.g., to remove fluid from polyps or edematous tissue) a hyperosmolar agent may be employed. Examples of suitable hyperosmolar agents include, but are not limited to, furosemide, sodium chloride gel, or other salt preparations that draw water from tissue or substances that directly or indirectly change the osmolar content of the mucous layer.

In an embodiment, immunomodulators may generally comprise immunosuppressants, immunostimulants, tolerogens, or combinations thereof. Examples of immunomodulators include imiquimod, cyclosporine, tacrolimus, azathioprine, cyclophosphamide, methotrexate, chlorambucil, mycophenolate mofetil (MMF), prednisolone, levamisole, and thalidomide.

Examples of leukotriene modifiers include, but are not limited to, montelukast.

Examples of mitotic inhibitors include, but are not limited to, mitomycin-C, taxanes, topoisomerase inhibitors, and vinca alkaloids.

Examples of muscle relaxants include cyclobenzaprine, dantrolene, metaxalone, and tizanidine.

Examples of mucolytics include, but are not limited to, acetylcysteine, domase alpha, guafenesin, or combinations thereof.

Examples of narcotic analgesics include, but are not limited to, codeine, fentanyl, hydrocodone, hydromorphone, meperidine, methadone, morphine, oxycodone, oxymorphone.

Examples of non-narcotic analgesics include, but are not limited to, acetaminophen, aspirin, celecoxib, diclofenac, ibuprofen, indomethacin, ketorolac, misoprostol, meloxicam, naproxen sodium.

Examples of vasoactive agents include, but are not limited to, nitrates, vasoconstrictors, vasodilators,

In an embodiment, the AI may be present in a suitable form. For example, in an embodiment, the AI may comprise a pharmaceutically acceptable salt, a suitable prodrug, a suitable solvate (e.g., a hydrous crystalline form an anhydrous crystalline form), or derivatives thereof, or combinations thereof.

As used herein, the term "pharmaceutically acceptable salt" refers to salts of active compounds which are prepared with relatively non-toxic acids. Acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic; propionic; isobutyric; maleic; malonic; benzoic; succinic; suberic; fumaric; mandelic; phthalic; benzenesulfonic; toluenesulfonic, including p-toluenesulfonic, m-toluenesulfonic, and o-toluenesulfonic; citric; tartaric; methanesulfonic; and the like. Also included are salts of amino acids such as arginate and the like, and salts of organic acids like glucuronic or galactunoric acids and the like (see, for example, Berge et al. J. Pharm. Sci. 66:1-19 (1977, which is incorporated herein by reference in its entirety)).

As used herein, the term "prodrug," refers to a compound that is a drug precursor which, upon administration to a subject, undergoes chemical conversion by metabolic or chemical processes to yield a desired compound (e.g., an API) or a salt and/or solvate thereof (e.g., a prodrug on being brought to the physiological pH or through enzyme action is converted to the desired drug form). A discussion of prodrugs is provided in T. Higuchi and V. Stella, Pro-drugs as Novel Delivery Systems (1987) Volume 14 of the A.C.S. Symposium Series, and in Bioreversible Carriers in Drug Design, (1987) Edward B. Roche, ed., American Pharmaceutical Association and Pergamon Press, both of which are incorporated in their entirety herein by reference.

As used herein, the term "solvate" refers to a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is water.

In an embodiment, the active ingredient may be present in the BAIESS in an amount of from about 0.01% to about 95%, from about 0.01% to about 90%, from about 0.01% to about 80%, from about 0.01% to about 70%, from about 0.01% to about 60%, from about 0.01% to about 50%, from about 0.01% to about 40%, from about 0.01% to about 30%, from about 0.01% to about 20%, from about 0.01% to about 10%, from about 0.01% to about 5%, from about 0.01% to about 1%, or from about 0.01% to about 0.25% by the total weight of the BAIESS. Not intending to be bound by theory, the amount of AI used may depend upon one or more factors including, but not limited to, the particular ingredient incorporated, any other AIs that are also incorporated, the condition or conditions being treated, the progression of the condition or conditions being treated, the age of the patient, the gender of the patient, the size of the patient, the intended site of deployment, the severity of clinical symptoms, the desired or approved dosage regimen, or combinations thereof.

In an embodiment, the AI may be eluted (e.g., released) from the BAIESS at a suitable rate. For example, the BAIESS may be configured to elute the AI at a rate, per day, in the range of from a first end point (A) to a second end point (B). For example, the BAIESS may be configured to elute the AI at a rate, per day, in the range of from (A) at least about 0.01 µg, alternatively, at least about 0.1 µg, alternatively, at least about 1 µg, alternatively, at least about 5 µg, alternatively, at least about 10 µg, alternatively, at least about 25 µg, alternatively at least about 50 µg, alternatively, at least about 75 µg, alternatively, at least about 100 µg, alternatively, at least about 150 µg, alternatively, at least about 200 µg, to (B) at most about 10 µg, alternatively, at most about 25 µg, alternatively, at most about 30 µg alternatively, at most about 40 µg, alternatively, at most about 50 µg, alternatively, at most about 75 µg, alternatively, at most about 100 µg, alternatively, at most about 200 µg, alternatively, at most about 300 µg, alternatively, at most to about 400 µg, alternatively, at most to about 500 µg, alternatively, at most to about 600 µg, alternatively, at most to about 700 µg, alternatively, at most to about 800 µg, alternatively, at most to about 900 µg, alternatively, at most to about 1,000 µg, alternatively, at most to about 1,250 µg, alternatively, at most to about 1,500 µg, alternatively, at most to about 2,000 µg, or at any other suitable rate. Not intending to be bound by theory, the rate of elution of the AI may depend upon one or more factors, examples of which include, but are not limited to those factors set forth above.

In an embodiment, the AI may be eluted from the BAIESS over a suitable period of time, for example, as may be at least partially dependent upon the intended use of the platform and/or the BAIESS. For example, in an embodiment the AI may be eluted from the BAIESS such that at least 50%, alternatively, at least 60%, alternatively, at least 70%, alternatively, at least 80%, alternatively, at least 90%, alternatively, at least 95%, alternatively, at least 97.5%, alternatively, at least 98%, alternatively, at least 99%, alternatively, at least 99.5%, alternatively, at least 99.9% of the AI associated with the BAIESS will be eluted therefrom within a suitable duration of time. For example, in an embodiment, the AI may be eluted to such a desired extent in a duration ranging from (A) a start point of not less than about 72 hours, alternatively, not less than about 96 hours, alternatively, not less than about 5 days, alternatively not less than about 7 days, alternatively, not less than about 14 days, alternatively, not less than about 21 days, alternatively, not less than about 28 days, alternatively, not less than about 1 month, alternatively, not less than about 2 months, alternatively, not less than about 3 months, alternatively, not less than about 4 months, alternatively, not less than about 6 months, alternatively, not less than about 8 months, alternatively, not less than about 10 months, alternatively, not less than about 12 months to (B) an end point of about not more than about 7 days, alternatively, not more than about 14 days, alternatively, not more than about 21 days, alternatively, not more than about 28 days, alternatively, not more than about 1 month, alternatively, not more than about 2 months, alternatively, not more than about 3 months, alternatively, not more than about 4 months, alternatively, not more than about 6 months, alternatively, not more than about 8 months, alternatively, not more than about 10 months, alternatively, not more than about 12 months, alternatively, not more than about 15 months, alternatively, not more than about 18 months, alternatively, not more than about 24 months.

In an embodiment, the duration for AI to be eluted may be less than, alternatively, substantially the same as, alternatively, the same as the duration over which the platform of the BAIESS degrades and/or biodegrades. In an embodiment, the elution of the AI may be contemporaneous with the degradation and/or biodegradation of the BAIESS. In an alternative embodiment, the elution of the AI may be partially contemporaneous with the degradation and/or biodegradation of the BAIESS. For example, the elution of the AI may begin substantially contemporaneous with, alternatively, after, biodegradation of the BAIESS has begun and/or, the elution of the AI may end substantially contemporaneous with, alternatively, before, biodegradation of the BAIESS has ended.

In various embodiments, the AI may be added to the platform in any suitable manner, for example, as will allow the AI to be eluted from or by the BAIESS, as will be disclosed herein. In an embodiment, the active agent may be coated onto the surface of the platform or a portion thereof. For example, the AI may be dissolved or suspended in a solution, which may be applied to one or more surfaces of the platform (e.g., by spraying, dipping, bathing, adsorption, absorption, or the like). Alternatively, the AI may be powder coated onto one or more surfaces of the platform and adhered thereto, for example, by heating the platform, softening the platform with a plasticizer, or by any suitable process.

In an additional or alternative embodiment, the AI may be incorporated into the platform or a portion thereof, for example, the AI may be incorporated throughout all portions of the device or in particular portions of the device. For example, in an embodiment the AI may be incorporated into the polymer composition (e.g., the degradable polymer) utilized to for the platform, for example, as disclosed herein. In such an embodiment, the AI may be dispersed (e.g., from the polymer matrix) as the degradable polymer degrades, for example, thereby releasing the incorporated AI. Additionally or alternatively, the platform may comprise a plurality of pores, each of the pores retaining AI, so as to act as a mechanical and/or physical barrier to release of the AI, for example, such that the AI may be released via erosion, diffusion, or combinations thereof, of the platform. Additionally or alternatively, the AI may be encapsulated and/or microencapsulated and the encapsulated/microencapsulated AI may be added to the platform (e.g., to the surface of the platform) or incorporated within the platform.

In an embodiment, the BAIESS may be configured such that the one or more AIs may be eluted differently with respect to time. For example, in an embodiment, a single AI may be added to the platform such that the AI is eluted at a rate that varies over time. In an embodiment, multiple AIs may be added to the BAIESS (e.g., the platform) such that the multiple AIs are eluted at independently variable rates. For example, one or more AIs may be added to the BAIESS in a plurality of layers, within pockets, in reservoirs, encapsulated in microspheres which may allow the release of the AI at variable rates, or combinations thereof. Additionally, in an embodiment, a portion of the AI may be quick-release (e.g., configured for immediate or substantially-immediate release from the BAIESS) and another portion of the AI may be extended release (e.g., configured for release, from the same or a difference portion of the BAIESS, over a relatively long duration of time).

Various embodiments of a BSS/BAIESS having been disclosed, one or more methods of utilizing such a BSS/BAIESS are also now disclosed. In an embodiment, the BSS/BAIESS may be utilized or used in the treatment of a patient. In an embodiment, a BSS/BAIESS is configured for use in treating a patient in need thereof. In an embodiment, the patient may be generally characterized as experiencing a dysfunction, undesirable medical condition, disorder, or disease state. The dysfunction, undesirable medical condition, disorder, or disease state will be collectively referred to hereinafter as an "undesirable condition." For example, the undesirable condition may include conditions such as "genetic diseases" which refer to conditions attributable to one or more gene defects. An "undesirable condition" may also include "acquired pathologies" which refer to pathological conditions that are not attributable to inborn defects, cancers, diseases, and the like. Examples of "acquired pathologies" include, but are not limited to, infectious diseases, post-operative states, or traumatic defects. Additionally, an "undesirable conditions" may include structural or functional conditions, for example, that develop throughout life, such as upper airway collapse during inspiration (e.g., as seen in obstructive sleep apnea), chronic Eustachian tube dysfunction, chronic rhinosinusitis, vertebral disk disease, presbyphonia, natural cosmetic aging, peripheral vascular disease, fertility beyond the timeframe of desired reproduction, Type II diabetes mellitus, morbid obesity, or the like. In an embodiment, the undesirable condition may comprise one or more of the conditions or states disclosed herein or any other condition or state as may be appreciated by one skill in the art upon viewing this disclosure.

Herein "treatment" refers to an intervention performed with the intention of preventing the development or altering the pathology of such an undesirable condition. Accordingly "treating" refers both to therapeutic treatments and to prophylactic measures. In an embodiment, administration of therapeutic amounts of compositions and/or at least one BSS/BAIESS (e.g., size, deployment site, number, of the BSS/BAIESS deployed) of the type described herein to an organism confers a beneficial effect on the recipient in terms of amelioration of the undesirable condition. Herein "therapeutic amounts" refers to the amount of the AI and/or at least one BAIESS necessary to elicit a beneficial effect. Alternatively, the compositions and/or at least one BAIESS described herein may be used prophylactically for reducing the potential onset or reoccurrence of an undesirable condition in a recipient not currently experiencing an undesirable condition.

For example, in an embodiment, the patient may be characterized as having been diagnosed with, experiencing symptoms associated with, wishing to prevent, or otherwise wishing to treat, medicate, correct, lessen the symptoms associated with, a disease, an illness, condition, an abnormality, a predisposition, and/or one or more of the symptoms associated therewith.

Additionally or alternatively, in an embodiment, the patient may be characterized as having recently undergone (or planning to undergo) a corrective procedure, a restorative procedure, an elective procedure, a cosmetic procedure, an urgent procedure, a life-extending procedure, a life-saving procedure, or combinations thereof, any of which may be undertaken or supplemental with a treatment of the type described herein (e.g., use of a BSS/BAIESS in furtherance thereof). For example, in an embodiment a BSS/BAIESS may be employed in ophthalmologic surgery, middle ear surgery, endoscopic sinus surgery, a surgery to improve airway collapse in obstructive sleep apnea, vocal cord surgery, tracheobronchial surgical procedures, gastrointestinal procedures or surgery, urologic surgery, gynecologic surgery for the enhancement or prevention of the likelihood of pregnancy, neurosurgery such as vertebral corrective procedures, endovascular surgery, cosmetic surgery, reconstructive surgery, a surgery to repair traumatic or burn defects, or combinations thereof.

In an embodiment, a method of utilizing or using a BSS/BAIESS may comprise administering the BSS/BAIESS to a patient in need thereof to treat an undesirable condition of the type(s) disclosed herein.

In an embodiment, a BSS/BAIESS may be deployed by a physician in a clinical setting. For example, in an embodiment a BSS/BAIESS may be deployed by the physician at, within, about, or otherwise proximate to an intended site of deployment within the patient where an undesirable condition exists. For example, in an embodiment, a BSS/BAIESS may be configured to be conformable to a lumen or other passageway. In such an embodiment, the BSS/BAIESS may be compressible, for example, radially compressible. In such an embodiment, the BSS/BAIESS may be compressed and/or contracted (e.g., radially compressed) loaded into a suitable deployment tool (e.g., a plunger or syringe-type deployment tool) configured to selectively retain the BSS/BAIESS in such a radially compressed conformation. The deployment tool may then be brought into the vicinity of the intended site of deployment (e.g., tissue), extruded or otherwise ejected or emptied from the deployment device, and allowed to radially expand. Alternatively, the BSS/BAIESS may be deployed utilizing one or more conventionally-utilized surgical instruments, such as forceps (e.g., bayonet forceps) and/or a speculum (e.g., a nasal speculum). For example, upon providing access to the site of deployment (e.g., with a speculum or other, similar device), the BSS/BAIESS may be brought into the vicinity of the intended site of deployment and released. Upon deployment, the BSS/BAIESS may contact the intended target tissue, for example, such that the propensity for radial expansion exhibiting by the BSS/BAIESS exerts a force so as to maintain patency of the lumen or other passageway.

In another embodiment, a BSS/BAIESS may be deployed by a physician in conjunction with an operative procedure. For example, using a syringe-like or plunger-like type of deployment tool or any suitable mode of deployment, a surgeon may bring the apparatus, previously having been loaded with a BSS/BAIESS, into the vicinity of the intended target tissue for treatment, and thus deploy the device, for example, during the course of an operative procedure. In an embodiment, a BSS/BAIESS that is conformable, trimmable, and malleable could be brought into the vicinity of a malignant tumor or the vascular inflow of such a malignant tumor, fashioned to conform to this structure, for example, so as both perform occlusive functions and (when an AI is present) targeted AI delivery to the intended treatment site. In another embodiment, a BSS/BAIESS may be deployed post-operatively. For example, in an embodiment, a BSS/BAIESS could be deployed into (e.g., allowed to radially expand within) a lumen or passageway, for example, to maintain postoperative patency for a defined period of time while (when an AI is present) simultaneously delivering AI(s) to aid in therapy and healing of the surgical site.

In still another embodiment, the BSS/BAIESS may be configured to not necessarily expand or contract, but rather provide predictable time-release of AI at an intended target site and/or provide a structural barrier. For example, as will be disclosed herein in greater detail, a BSS/BAIESS may be deployed as an intrauterine device that could release contraceptive hormones, pro-fertility agents such as clomiphene, inhibit implantation of a fertilized egg into the endometrium, or any combination of the above. In such embodiments, as disclosed herein, after the period of time of usefulness, the BSS/BAIESS may be configured to biodegrade via the body's natural degradatory mechanisms.

In an embodiment, a BSS/BAIESS may be deployed within a lumen or other passageway. For example, a BSS/BAIESS may be deployed within a lumen or other passageway for the purpose of providing and/or maintaining patency of such lumen or passageway. In such an embodiment, the BSS/BAIESS may be deployed within the external auditory canal, the Eustachian tube, the nasolacrimal system, the upper or lower airway, the esophagus, the gastrointestinal tract, the hepatobiliary tract, the pancreatic duct, the genitourinary tract including the male and female reproductive tracts, intravascularly, inter-vertebrally, within the spinal canal or nerve foramena, at the anastamotic site of a vascular repair such as in a vascular bypass procedure or a microvascular free flap tissue transfer procedure, or in combinations thereof.

In an alternative embodiment, a BSS/BAIESS may be deployed around a tissue or other anatomical structure. For example, a BSS/BAIESS may be deployed around a tissue or other anatomical structure for example, for the purpose of delivering one or more AIs to the anatomical structure, or in the case of a vascular structure, to the anatomical structures downstream from it, for the purpose of occluding partially or completely this tissue, or combinations thereof. For example, in an embodiment, a deployment apparatus could be used by a surgeon to narrow the lower esophagus or the lower ureter in undesirable conditions such as gastroesophageal reflux disease or vesicoureteral reflux disease, respectively. In another embodiment, such a BSS/BAIESS could be used for complete, permanent occlusion of an anatomical structure, such as the Fallopian tube, to prevent an egg from reaching the uterus for possible fertilization, in the vas deferens for prevention of sperm reaching the eventual semen and ejaculate, or in the feeding vessel of an aneurysm or varicosity to completely prevent vascular inflow.

Alternatively, in an embodiment, a method of utilizing a BSS/BAIESS may comprise making a BSS/BAIESS available to a patient, for example, for deployment by the patient themselves and/or by another (e.g., a parent or caregiver) in a non-clinical setting. In such an embodiment, For example, in an embodiment, a BSS/BAIESS may be configured for deployment by a patient or another. For example, in an embodiment, the patient or another (e.g., a non-physician) may obtain a BSS/BAIESS deployment device. For example, the BSS/BAIESS may be compressed within (e.g., preloaded and/or packaged) such a deployment device such that, the patient themself may deploy the BSS/BAIESS within a suitable lumen, passageway, or the like. Not intending to be bound by theory, the BSS/BAIESS and/or the deployment device may be configured for safe deployment by the patient themselves, for example, without concern that the BSS/BAIESS will be deployed wrongly, unsafely, or inappropriately.

In an additional embodiment, making the BSS/BAIESS available to a patient may comprise packaging the BSS/BAIESS with instructions, illustrations, or the like, depicting deployment procedures. Also, in an embodiment, making the BSS/BAIESS available to a patient may comprise packaging the BSS/BAIESS with a deployment device (e.g., a preloaded plunger-like or syringe-like device), for example, allowing for deployment by the patient and/or another.

### Particular Embodiments

One or more embodiments of the BSS/BAIESS and methods of utilizing the same having been generally disclosed, the following particularized embodiments of the BSS/BAIESS and methods of using such a BSS/BAIESS are also disclosed. Although the following embodiments may set forth particular embodiments of the BSS/BAIESS and/or particular methods of utilizing that particular BSS/BAIESS, this disclosure should not be construed as limiting the disclosed embodiments of a BSS/BAIESS to any particular method of utilization, nor should any particular method of utilizing a BSS/BAIESS be limited to any particular BSS/BAIESS.

### Nasal Splint Embodiments

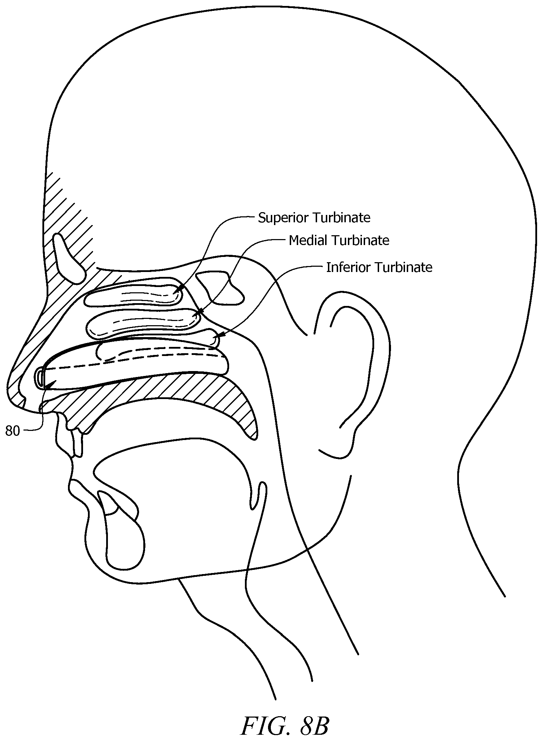

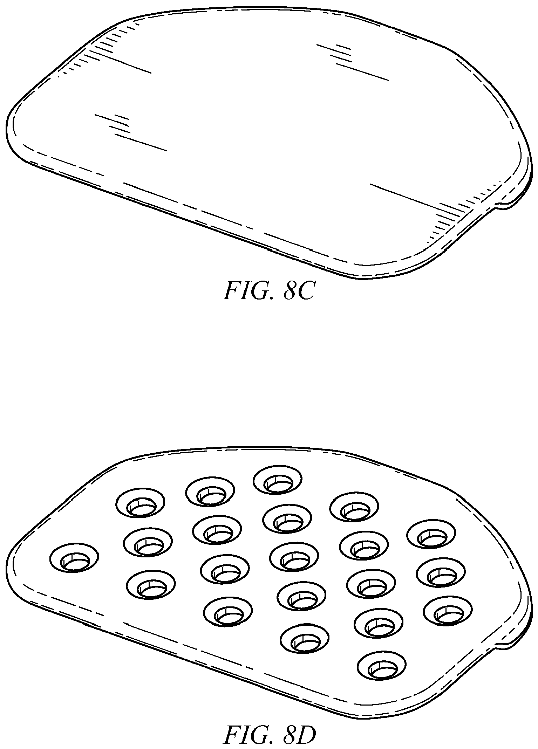

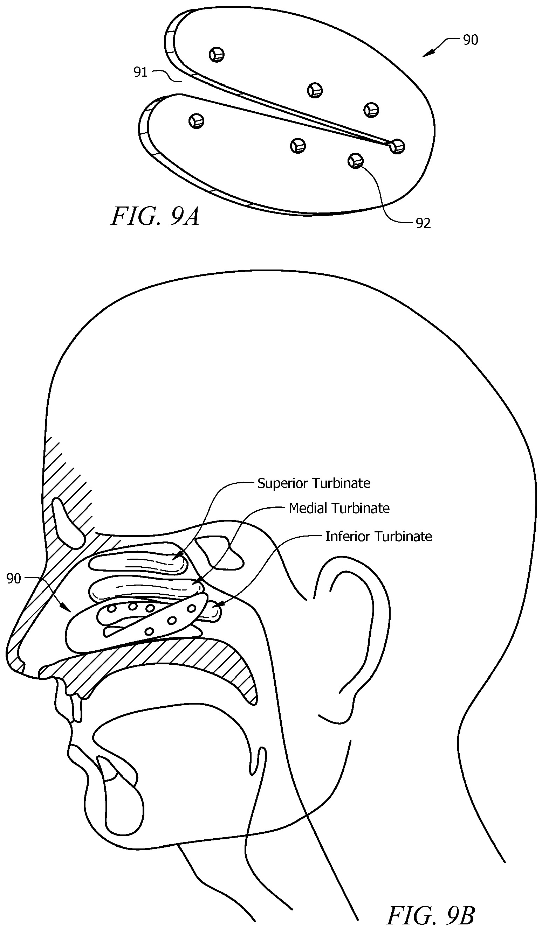

In an embodiment, the BSS/BAIESS comprises, is comprised of, and/or is configured to be effective as a nasal splint. In such an embodiment, such a BSS/BAIESS may be configured for placement within and/or at least partially within the nasal cavity, for example, to stabilize a surgically-straightened nasal septum. Referring to Figure 8A, a first embodiment of a BAIESS comprising a nasal splint 80 is illustrated. In the embodiment of Figure 8A, the nasal splint (e.g., illustrated in a deployed state) may be characterized as similar, for example similar in form and/or structure, to a Doyle-type nasal splint. In the embodiment of Figure 8A, the nasal splint 80 generally comprises a hollow, cylindrical (e.g., a tube-like) component 82 and may further comprise a flap 81. In an embodiment, as will be appreciated by one of skill in the art upon viewing this disclosure, such a nasal splint may be configured for placement in either the left or the right nasal cavity and may be sized accordingly, for example, having a diameter and/or a length sized for placement within the nasal cavity. For example, referring to Figure 8B, the nasal splint 80 is illustrated deployed within the nasal cavity of a patient. In the embodiment of Figure 8B, the nasal splint 80 is positioned substantially adjacent to the nasal septum and the anterior and medial edges of the inferior turbinate. Additionally, in the embodiment of Figure 8B, the nasal splint 80 is deployed such that the flap 81 abuts the nasal septum and such that the hollow, cylindrical component 82 abuts the medial surface of the inferior turbinate. In an embodiment, the BSS/BAIESS may rest along the floor of the nasal cavity. Referring to Figures 8C, 8D, and 8E, various embodiments of a sheet (e.g., which may be rolled, so as to form the nasal splint 80) are illustrated. Various embodiments of such a nasal splint are disclosed in Application Serial No. 29/432,554, filed September 17, 2012, which is incorporated herein in its entirety by reference. Referring to Figure 9A, a second embodiment of a BSS/BAIESS comprising a nasal splint 90 is illustrated. In the embodiment of Figure 9A, the nasal splint (e.g., in a deployed state) may be characterized as similar, for example in form and/or structure, to a Reuter bivalve-type nasal splint. In the embodiment of Figure 9A, the nasal splint generally comprises a lengthwise slit 91 and a plurality of perforations or holes 92 (e.g., spaces between strands or filaments).

In either of such embodiments, a BSS/BAIESS comprising a nasal splint may be configured to exert a force, for example, a radially outward force, upon deployment within the nasal cavity, for example to stabilize (e.g., medialize) a surgically-corrected (e.g., surgically-straightened) nasal septum and/or to simultaneously lateralize the inferior turbinate (e.g., to exert a lateralizing force against the inferior turbinate), for example, after outfracture, cauterization, radiofrequency ablation, submucous resection, the like, or combinations thereof. For example, a BSS/BAIESS comprising a nasal splint may be configured to provide such a radially outward force to provide septal support, and/or to reduce or prevent stenosis of the nasal cavity. For example, in the embodiment of Figures 8A and 8B, the nasal splint comprising a hollow cylinder and flap comprising the nasal splint may be radially compressed for placement within the nasal cavity (e.g., the flap may be rolled over and/or partially around the hollow cylinder and, optionally, held in place with one or more biodegradable bands such as biodegradable, elastomeric bands). Similarly, in the embodiment of Figures 9A and 9B, the nasal splint comprising a slit may be compressed inward (e.g., a first portion overlapped over a second portion via the slit such that the nasal splint becomes more narrow) for placement within the nasal cavity. In either of such embodiments, the nasal splint may be allowed to expand radially outward, for example, so as to apply a physical force to the nasal septum and/or the inferior turbinate upon placement and deployment within the nasal cavity.

In an embodiment, a BAIESS comprising a nasal splint may comprise a suitable AI or combination of AIs. Alternatively, a suitable AI or combination of AIs may be applied to a BSS comprising a nasal splint. As will be disclosed herein, the AI and/or combination of AIs may be at least partially dependent upon the patient and/or the conditions experienced by the patient.

In an embodiment, a method of utilizing a BSS/BAIESS comprising a nasal splint may comprise deploying the BSS/BAIESS comprising the nasal splint within the nasal cavity of a patient. In an embodiment, such a patient may be characterized as having recently undergone a septoplasty and/or an inferior turbinate reduction, as suffering from (e.g., experiencing one or more signs, symptoms, or the like, associated with) allergic rhinitis, viral rhinitis, rhinosinusitis of viral or bacterial or unknown origin, and/or vasomotor (non-allergic) rhinitis, as suffering from an external and/or internal nasal valve collapse (e.g., as may result from exertion during inspiration, such as during exercise), as suffering from snoring and/or obstructive sleep apnea (e.g., as may result from an external or internal nasal valve collapse), or combinations thereof.

In an embodiment where the BSS/BAIESS comprising a nasal splint may be deployed within the nasal cavity of a patient characterized as having recently undergone a septoplasty and/or an inferior turbinate reduction. When present, the AI may confer a therapeutic benefit upon the tissue at the site of deployment (e.g., the operative site). In an embodiment where the AI is present, the AI may comprise a corticosteroid such as beclomethasone, budesonide, ciclesonide, fluticasone furoate, fluticasone propionate, flunisolide, mometasone, or triamcinolone, an antihistamine such as azelastine or olopatadine, an anticholinergic agent such as ipratroprium bromide, an antibiotic such as, but not limited to, doxycycline, mupirocin, trimethoprim/sulfamethoxasole or combinations thereof. In an embodiment where the BSS/BAIESS comprising a nasal splint may be deployed within the nasal cavity of a patient characterized as suffering from allergic rhinitis, viral rhinitis, rhinosinusitis of viral or bacterial or unknown origin, and/or vasomotor (non-allergic) rhinitis, (when present) the AI may comprise a corticosteroid such as beclomethasone, budesonide, ciclesonide, fluticasone furoate, fluticasone propionate, flunisolide, mometasone, or triamcinolone, a topical antihistamine such as azelastine or olopatadine, an anticholinergic agent such as ipratroprium bromide, an antibiotic (such as, but not limited to, mupirocin, doxycycline, trimethoprim/sulfamethoxasole, levofloxacin) or combinations thereof. In an embodiment where the BSS/BAIESS comprising a nasal splint may be deployed within the nasal vestibule or nasal cavity of a patient characterized as suffering from an external and/or internal nasal valve collapse (e.g., as may result from exertion during inspiration, such as during exercise), there may be no AI, or the AI may comprise an antibiotic such as, but not limited to, mupirocin, doxycycline, trimethoprim/sulfamethoxasole, levofloxacin, or combinations thereof. In an embodiment where the BSS/BAIESS comprising a nasal splint may be deployed within the nasal vestibule or nasal cavity of a patient characterized as suffering from snoring and/or obstructive sleep apnea (e.g., as may result from an external or internal nasal valve collapse), a corticosteroid such as beclomethasone, budesonide, ciclesonide, fluticasone furoate, fluticasone propionate, flunisolide, mometasone, or triamcinolone, a topical antihistamine such as azelastine or olopatadine, an anticholinergic agent such as ipratroprium bromide, and antibiotic such as, but not limited to, mupirocin, or combinations thereof.

In an embodiment, a BSS/BAIESS comprising a nasal splint may be deployed by a physician. Referring to Figures 8B and 9B, an embodiment of a BSS/BAIESS comprising a nasal splint deployed within the nasal passage of a patient is illustrated. For example, the BSS/BAIESS comprising a nasal splint may be deployed within the nasal passage so as to lateralize at least a portion of the inferior turbinate. For example, the BSS/BAIESS comprising a nasal splint may exhibit mechanical activity so as to exert a force against the inferior turbinate effective to cause at least a portion of the inferior turbinate to be displaced laterally and/or to the side, and thereby increase the diameter of the airway. In an embodiment a BSS/BAIESS comprising a nasal splint may be deployed by utilization of a suitable plunger or syringe-type deployment tool, alternatively, via any suitable mode of deployment. In an embodiment, the BSS/BAIESS comprising a nasal splint will be compressible, for example, radially compressible. In such an embodiment, the BSS/BAIESS may be compressed and/or contracted (e.g., radially compressed) loaded into the plunger or syringe-type deployment tool. The deployment tool embodiment may then be brought into the vicinity of the intended site of deployment (e.g., tissue), extruded or ejected from the deployment device, and allowed to radially expand, for example, thereby allowing for contact with the intended target tissue. Alternatively, the BSS/BAIESS may be deployed, as disclosed herein, utilizing conventionally-utilized surgical instruments.

In an embodiment, a BSS/BAIESS comprising a nasal splint may be characterized as exhibiting at least about 95% degradation in a duration of from about 5 days to about 45 days, alternatively, from about 7 days to about 30 days, alternatively, about 7 to 10 days, alternatively, about 28 to 31 days. In an embodiment, a BAIESS comprising a nasal splint may be characterized as exhibiting at least about 95% elution of the AI in a duration of about 5 days to about 45 days, alternatively, from about 7 days to about 30 days, alternatively, about 7 to 10 days, alternatively, about 28 to 31 days.

As may be appreciated by one of skill in the art upon viewing this disclosure, nasal splints are used frequently, often in a postoperative setting. A BSS/BAIESS comprising a nasal splint, for example, as disclosed herein, offers significant advances over the existing art. For example, because the BSS/BAIESS degrades/biodegrades, uncomfortable removal procedures are avoided. Also, by delivering active ingredients at the surgical site, which current devices do not allow for, the BSS/BAIESS, when utilized postoperatively, may dramatically speed up healing and minimize scarring, as well as, lessen or eliminate the costs and risks of systemic side effects associated with an oral prescription antibiotic and/or corticosteroid. Further, a BSS/BAIESS comprising a nasal splint may provide significant therapeutic benefit. For example, such a BSS/BSIESS may allow for targeted delivery of an AI (e.g., a therapeutic agent, such as a steroid) into the nasal cavity. In such an embodiment, the targeted delivery of such a therapeutic agent may yield a substantial decrease in any obstructions within the nasal passage, for example, by decreasing the size of the inferior turbinate. The therapeutic agent may also prevent scar formation, and/or reduce the likelihood of revisionary surgery.

### Over-the-Counter Respiratory Device Embodiments

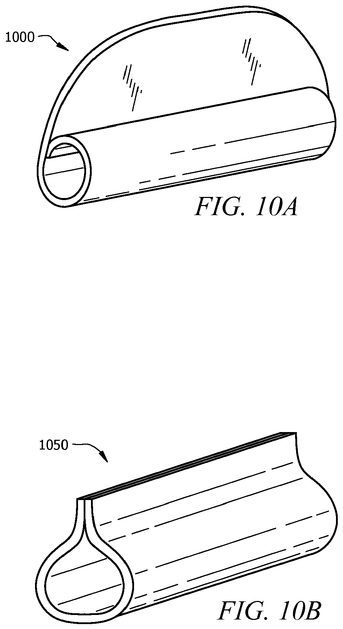

In an embodiment, the BSS/BAIESS comprises, is comprised of, and/or is configured to be effective as an over-the-counter (OTC) Respiratory Device. In such an embodiment, the OTC Respiratory Device 100 may be configured for placement within and/or at least partially within the nasal cavity. Referring to Figure 10A, a first embodiment of such an OTC Respiratory Device is illustrated. In the embodiment of Figure 10A, the BSS/BAIESS may be characterized as similar in form and/or structure to a Doyle-type nasal splint, but having a length being substantially shorter, for example, at least 0.5cm shorter, alternatively, at least 1 cm shorter, alternatively, at least 1.5 cm shorter. In such an embodiment, the OTC Respiratory Device 1000 may be sized, for example, having a diameter and/or a length such that the device is suitable for placement within the nasal vestibule, in a particular embodiment, anterior to the leading edge of the inferior turbinate.

Referring to Figure 10B, a second embodiment of such an OTC Respiratory Device is illustrated. In the embodiment of Figure 10B, the BSS/BAIESS generally comprises a hollow, generally tubular structure. In the embodiment of Figure 10B, the OTC Respiratory Device 1050 may be characterized as having a "tear-drop" shaped cross-section (e.g., perpendicular to the longitudinal axis. Similarly, the OTC Respiratory Device 1050 may also be sized, for example, having a diameter and/or a length such that the device is suitable for placement within the nasal vestibule, in a particular embodiment, anterior to the leading edge of the inferior turbinate

In an embodiment, such a BSS/BAIESS comprising an OTC Respiratory Device, such as OTC Respiratory Devices 1000 and/or 1050, may be configured for deployment by a patient without the aid of a healthcare provider. In such an embodiment, such a BSS/BAIESS may be suitable for the treatment of an acute, upper respiratory illness. Not intending to be bound by theory, and as will be disclosed herein, such a BSS/BAIESS comprising a nasal splint may be configured for safe deployment by a patient (e.g., in a non-clinical setting) in that the configuration does not allow deployment too deep within the nasal cavity (e.g., within an unsafe and/or undesired portion).

In an embodiment, a BSS/BAIESS comprising an OTC Respiratory Device may be suitably utilized by a patient in the treatment of sleep apnea, snoring, acute upper respiratory illnesses, such as the common cold, bacterial rhinosinusitis, allergic rhinitis and/or one or more of the symptoms associated therewith. For example, in an embodiment a patient may obtain a BAIESS containing a protein, such as an allergen or allergens intended for immunotherapy (e.g., pollen), a corticosteroid such as beclomethasone, budesonide, ciclesonide, fluticasone furoate, fluticasone propionate, flunisolide, mometasone, or triamcinolone, a topical antihistamine such as azelastine or olopatadine, an anticholinergic agent such as ipratroprium bromide, and antibiotic such as, but not limited to, mupirocin, or combinations thereof.

In an additional or alternative embodiment, a BSS/BAIESS comprising an OTC Respiratory Device may be suitably utilized by a patient in inhibiting or preventing an overgrowth, for example, by one or more *Staphylococcus* species, with resultant propagation of methicillin resistant *Staphylococcus aureus* (MRSA) from one colonized patient to another, or even toxic shock syndrome. As will be appreciated by one of skill in the art upon viewing this disclosure, limitation of the spread of MRSA is a significant public health and hospital initiative.

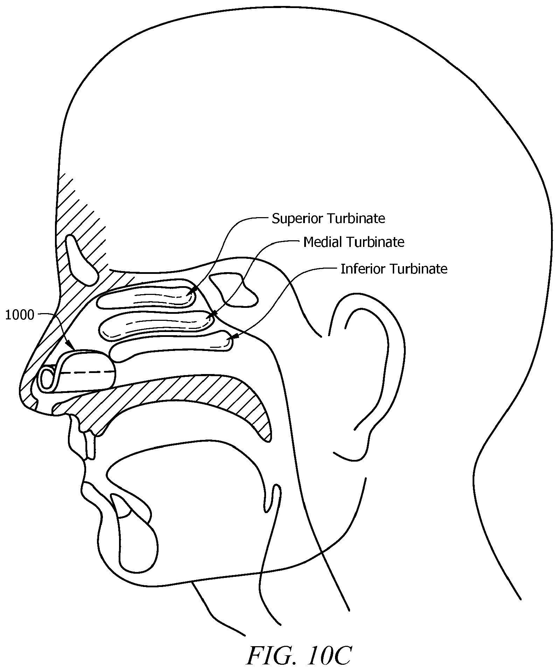

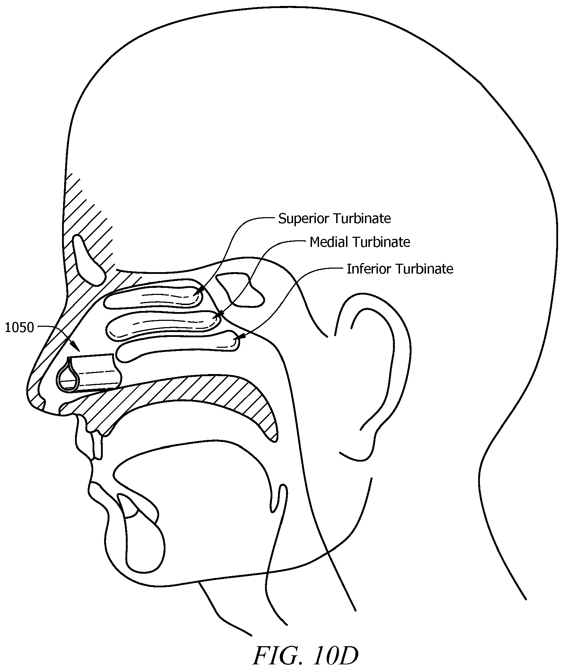

In alternative an embodiment, the BSS/BAIESS may be configured for placement in a non-clinical setting, for example, by the patient themselves. For example, in an embodiment, a BSS/BAIESS comprising an OTC Respiratory Device may be configured for deployment by a patient. Referring to Figures 10C and 10D, embodiments of a BSS/BAIESS comprising an OTC Respiratory Device, such as OTC Respiratory Devices 1000 and/or 1050, are illustrated deployed within the nasal passage of a patient. For example, in an embodiment, the patient may obtain the BSS/BAIESS comprising a nasal splint component along with deployment device, for example, compressed within the deployment device such that, the patient themself may deploy the BSS/BAIESS within the nasal vestibule anterior to the leading edge of the inferior turbinate. Not intending to be bound by theory, the BSS/BAIESS and/or the deployment device may allow for safe deployment by the patient themselves, in the treatment of an acute upper respiratory illness, for example, without concern that the BSS/BAIESS will be deployed too deep within the nasal cavity. In an additional embodiment, the BSS/BAIESS may be packaged with instructions, illustrations, or the like, depicting placement of the BSS/BAIESS comprising the OTC Respiratory Device by the patient.

In an embodiment, a BSS/BAIESS comprising an OTC Respiratory Device may be characterized as exhibiting at least about 95% degradation in a duration of from about 24 hours to about 7 days, alternatively, from about 2 days to about 5 days, alternatively, about 3 days to about 5 days. In an embodiment, a BSS/BAIESS comprising an OTC Respiratory Device may be characterized as exhibiting at least about 95% elution of the AI in a duration of from about 24 hours to about 7 days, alternatively, from about 2 days to about 5 days, alternatively, about 3 days to about 5 days.

While presently-available devices may be configured to provide physically support or hold open nasal passages, such conventional devices do little or nothing to prevent other problems that may occur resultant to abnormalities of the nasal passageways. For example, Breathe-Right Strips®, which may be used on the outside of the nose, may help to correct a valve collapse and/or to assist with proper respiration during sleep or exercise. Nonetheless, in the situations of snoring, sleep apnea, and/or an internal or external valve collapse, the possibility of overgrowth by one or more *Staphlyococcus* species, with resultant toxic shock syndrome, due to the presence and duration of such *Staphylococci,* remains a threat. In an embodiment, a BAIESS comprising an OTC Respiratory Device, for example, as may be deployed in a non-clinical setting, may comprise an AI such as doxycycline, mupirocin, trimethoprim/sulfamethoxasole, or the like, as described herein to limit to possibility of *Staphylococci* overgrowth and the risks associated therewith while contemporaneously improving the patency of the nasal passageways.

Additionally, a BSS/BAIESS comprising an OTC Respiratory Device, for example, as may be deployed in a non-clinical setting, may be advantageously employed in the treatment of acute, upper respiratory illness, for example, by both providing a physical means for improving the patency of nasal passages while (when an AI is present) eluting an AI effective for the treatment of that illness substantially proximate to the site of the illness and for an extended duration. As such, not intending to be bound by theory, such a two-pronged method of treatment may improve the condition of patients suffering from such an upper respiratory illness with improved efficacy in comparison to conventional means of treatment.

Additionally, a BSS/BAIESS comprising an OTC Respiratory Device, for example, as may be deployed in a non-clinical setting, may be advantageously employed in a desensitization process associated with immunotherapy in a patient with allergic rhinitis. For example, a BSS/BAIESS may provide both a physical means for improving the patency of nasal passages while at least partially contemporaneously instilling the allergen(s) in the patient's nasal airway at such a dose and time schedule so to allow the patient to develop tolerance to one or more allergens, for example, similar to the fashion of currently injected immunotherapy (e.g., "allergy shots") or sublingually instilled immunotherapy (e.g., "allergy drops," for under the tongue usage). As such, not intending to be bound by theory, such a two-pronged method of treatment may improve the condition of patients suffering from allergic rhinitis, for example, by maintaining nasal patency while providing equal efficacy to conventional immunotherapy.

### Myringotomy Tube Embodiments

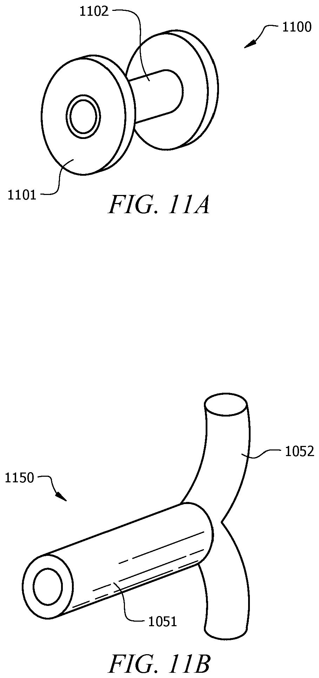

In an embodiment, the BSS/BAIESS comprises, is comprised of, and/or is configured to be effective as a myringotomy tube (also referred to as a tympanostomy tube, a grommet tube, a pressure-equalization tube, a PE tube, or an ear tube). In such an embodiment, such a BSS/BAIESS may be configured for placement within and/or into the tympanic membrane, for example, to provide for ventilation of the middle ear in the absence of normal Eustachian tube function (e.g., to prevent or inhibit the accumulation of fluid or negative air pressure within the middle ear). Referring to Figure 11A, a first embodiment of a BSS/BAIESS comprising a myringotomy tube is illustrated. In the embodiment of Figure 11A, the myringotomy tube may be characterized as similar, for example, similar in form and/or structure, to an Armstrong grommet-type tube. In an embodiment, such an Armstrong-type BSS/BAIESS 1100 may comprise a hollow, generally cylindrical, solid structure 1102, optionally having flange-like projections 1101 toward the ends thereof (e.g., a grommet). In an embodiment, such an Armstrong-type BSS/BAIESS may have an outside diameter of from about 2 mm to about 3 mm, a bore (e.g., opening) having a diameter of from about 1 mm to about 2 mm, and a length of from about 2 mm to about 3 mm. In an embodiment, one or both ends of the Armstrong-type BSS/BAIESS may be beveled.

Referring to Figure 11B, a second embodiment of a BSS/BAIESS comprising a longer-standing "T-tube" is illustrated. In the embodiment of Figure 11B, this longer-standing myringotomy tube (e.g., in a deployed state) may be characterized as similar, for example, similar in form and/or structure, to a Richards-type tube (e.g., a "T-tube"). In an embodiment, such a Richards-type BAIESS 1150 may comprise a hollow, generally cylindrical solid structure 1151, optionally having a flange-like projection toward one end and a second hollow, generally cylindrical structure 1152 intersecting the other end so as to allow fluid communication or ventilation of air between the two hollow bores defined by the two structures. In an embodiment, such a Richards-type BSS/BAIESS may have an outside diameter of from about 2 mm to about 3 mm, a bore (e.g., opening) having a diameter of from about 1 mm to about 2 mm, and a length of from about 10 mm to about 20 mm. In an embodiment, additional or alternative configurations and/or variations of the configurations of a BSS/BAIESS comprising a myringotomy tube may be appreciated by one of skill in the art upon viewing this disclosure.

In either of such embodiments, a BSS/BAIESS comprising a myringotomy tube may be generally configured to prevent unintended closure (e.g., healing) of the tympanic membrane and/or to provide structural support to the tympanic membrane, for example, thereby allowing for aeration (e.g., ventilation or draining) of the middle ear. For example, in the embodiments of Figures 11A and 11B, the BSS/BAIESS comprising an Armstrong-type and/or a Richards-type myringotomy tube each define a bore allowing for aeration which the BSS/BAIESS comprising a myringotomy tube structurally prevents closure (e.g., unintended healing) of the tympanic membrane.

Additionally, in an embodiment, a BAIESS comprising a myringotomy tube may comprise a suitable AI or combinations of AIs. As will be disclosed herein, the AI and/or combination of AIs may be at least partially dependent upon the patient and/or the conditions experienced by the patient. For example, in an embodiment, the AI may comprise a corticosteroid such as beclomethasone, budesonide, ciclesonide, fluticasone furoate, fluticasone propionate, flunisolide, mometasone, or triamcinolone, an antihistamine such as azelastine or olopatadine, an anticholinergic agent such as ipratroprium bromide, an antibiotic such as, but not limited to, mupirocin, ciprofloxacin, ofloxacin, a growth-stimulating factor or factors (e.g., to promote tympanic membrane healing after biodegradation/extrusion of tube), or combinations thereof.

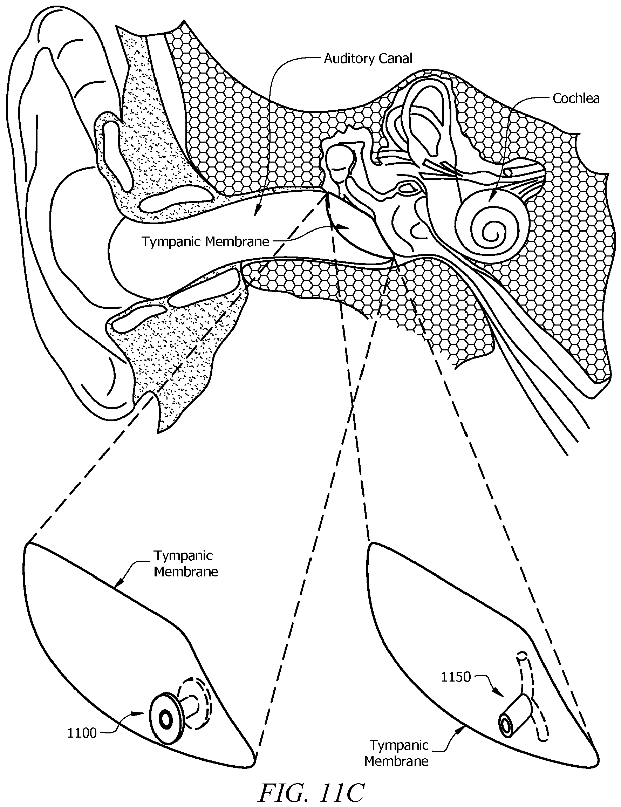

In an embodiment, a method of utilizing a BSS/BAIESS comprising a myringotomy tube may comprise deploying the BSS/BAIESS comprising the myringotomy tube within the tympanic membrane (e.g., within an incision within the tympanic membrane) of a patient. Referring to Figure 11C , embodiments of a BSS/BAIESS comprising a myringotomy tube (e.g., the BSS/BAIESS comprising a myringotomy tube of Figures 11A and 11B) deployed within the tympanic membrane of a patient is illustrated. In an embodiment, such a patient may be characterized as suffering from recurrent acute otitis media, chronic serous otitis media, chronic serous otitis media with effusion, chronic suppurative otitis media, acute mastoiditis, chronic mastoiditis, Eustachian tube dysfunction, tympanic membrane retraction, cholesteatoma, conductive hearing loss, mixed conductive and sensorineural hearing loss, or combinations thereof.

In an embodiment, a BSS/BAIESS comprising a myringotomy tube may be deployed by a physician. For example, in an embodiment a BAIESS comprising a myringotomy tube may be deployed by a physician a during a myringotomy, for example, a surgical procedure in which an incision is created within the tympanic membrane, either conventionally with a myringotomy knife, with the use of laser equipment, or by future, yet-undeveloped, techniques as may be suitable for tube deployment within the tympanic membrane.

In an embodiment, a BSS/BAIESS comprising a myringotomy tube, for example, as disclosed herein, may have numerous advantages in comparison to conventional products in the marketplace, while offering many or all of the benefits associated with such conventional products. As may be appreciated by one of skill in the art upon viewing this disclosure, the biodegradability associated with such a BSS/BAIESS may eliminate concerns associated with leaving a foreign body within the ear, particularly, the external auditory canal, the tympanic membrane, the middle ear space, and/or the Eustachian tube. For example, in some patients, conventional myringotomy tubes do not extrude on their own volition, necessitating a second surgical procedure for tube removal and patching/grafting of the existing perforation within the tympanic membrane where the tube previously resided. Also, in some other patients, conventional myringotomy tubes may extrude inwardly into the middle ear space. Still further, in some patients, conventional myringotomy tubes may extrude properly into the external auditory canal, but may become encased in cerumen. In both instances, secondary office procedures and/or operative interventions are required for tube removal. Such improper extrusion of such a conventional myringotomy tube, particularly, in the case of extrusion medially into the middle ear, poses a risk of long-term damage to the ossicular chain (the bones responsible for sound transmission into the cochlea or inner ear) and/or the foreign body may serve as a nidus for infection or cholesteatoma formation. Finally, even with proper timing and positioning of the tube extrusion, the residual perforation in the tympanic membrane may persist beyond the intended duration, thereby necessitating an additional surgical procedure, for example to patch and/or graft the perforation. In an embodiment, because of the biodegradability associated with such a BAIESS comprising a myringotomy tube, these risks are mitigated, if not avoided entirely. Particularly, because a BSS/BAIESS comprising a myringotomy tube degrades over time, the necessity for secondary procedures or interventions is mitigated greatly in comparison to conventional myringotomy tubes.

In an additional embodiment, because a BAIESS comprising a myringotomy tube provides the capability to deliver an AI or multiple AI's using the BAIESS, for example, varying as to the AI(s) and/or the rate of elution of such AI(s) with respect to time, such a BAIESS comprising a myringotomy tube may allow for the opportunity to improve the therapeutic value by delivering therapeutic molecules substantially proximately, if not precisely, at the site where such therapeutic molecules are needed, for example, to combat infection and/or inflammation, to decrease the risk of residual tympanic membrane perforation after biodegradation or extrusion of the tube by stimulating healing of the membrane, or combinations thereof. For example, not intending to be bound by theory, incorporation of one or more AIs within a BAIESS, such as a BAIESS comprising a myringotomy tube, may decrease, or avoid entirely, the costs and/or side effects potentially associated with instillation of other pharmaceuticals. Also, the typical water precautions necessary when conventional myringotomy tubes are placed (e.g., ear plugs during showering and swimming, for example, to prevent bacteria in the water from resulting in an infection within the middle ear) could be mitigated and/or eliminated entirely in that the AI may sufficiently reduce or eradicate a population of pathogens which could result in such an infection as those pathogens move through the lumen (e.g., flowbore) of the BAIESS tube, prior to such pathogens ever reaching the middle ear space.

### Multi-Use Sheet Embodiments

In an embodiment, the BSS/BAIESS comprises, is comprised of, and/or is configured to be effective as a multi-use sheet. For example, such a multi-use BSS/BAIESS may comprise a conformable, sizable (e.g., by trimming) sheet or like material. In such an embodiment, such a BSS/BAIESS comprising a multi-use sheet may be configured for and/or configurable for placement within any suitable tubular or anatomic structure with a potential or actual space, for example, a lumen, duct, passageway, or the like and which may contain air and/or fluid. Examples of such a structure include, but are not limited to, the external auditory canal, the middle ear space, the Eustachian tube, the lacrimal duct, the pharynx, the larynx, the esophagus, the spinal canal, the vertebral foramen (e.g., around an exiting nerve), the tracheobronchial tree, the hepatobiliary tract, a pancreatic duct, the ureter, the urethra, a Fallopian tube, the uterus, the vagina, the vas deferens, a vascular anastamose (e.g., in the context of vascular surgery or microvascular free tissue transfer, such as a skin graft), or combinations thereof.

In an embodiment, such a multi-use BSS/BAIESS may be configured to maintain the patency of the tubular or other anatomic space internally (e.g., from the inside of a lumen or other anatomical space) and/or to at least partially concurrently deliver the AI(s) in a precise, local manner. For example, in an embodiment, a BSS/BAIESS comprising a multi-use sheet may be configured to be rolled and, when such a sheet is rolled, the multi-use sheet may be configured so as to exhibit a tendency to unroll, for example, so as to exhibit a radially outward force. As such, such a BSS/BAIESS comprising a multi-use sheet may be characterized as radially expandable, for example, so as to expand radially outward (e.g., unroll) when not retained in a rolled conformation. In such an embodiment, the BSS/BAIESS comprising a multi-use sheet may be configured to apply a radially-outward force (for example, so as to hold open or otherwise support a lumen, passageway, or other opening) upon deployment. For example, such a BSS/BAIESS comprising a multi-use sheet may be configured to be rolled (e.g., inwardly compressed) and deployed within a lumen or other passageway so as to unroll therein (e.g., exert an outward bias).

In an alternative embodiment, the BSS/BAIESS may be configured to compress or occlude the patency of an anatomic space, for example, externally, in an embodiment, at least partially concurrently with the delivery of one or more AI(s). For example, in an embodiment, a BSS/BAIESS comprising a multi-use sheet may be configured such that the multi-use sheet exhibits a tendency to roll, for example, so as to exhibit a radially inward force (e.g., a centripetal force). As such, such a BSS/BAIESS comprising a multi-use sheet may be characterized as radially contractible, for example, so as to contract radially inward (e.g., roll) when not retained in an unrolled and/or partially unrolled conformation. In such an embodiment, the BSS/BAIESS comprising a multi-use sheet may be configured to apply a radially-inward force (for example, so as to constrict a lumen, passageway, or other opening) upon deployment. For example, such a BAIESS comprising a multi-use sheet may be configured to be unrolled (e.g., outwardly expanded) and deployed around a lumen or other tissue (e.g., a vessel) so as to roll or otherwise constrict (e.g., exert an inward bias).

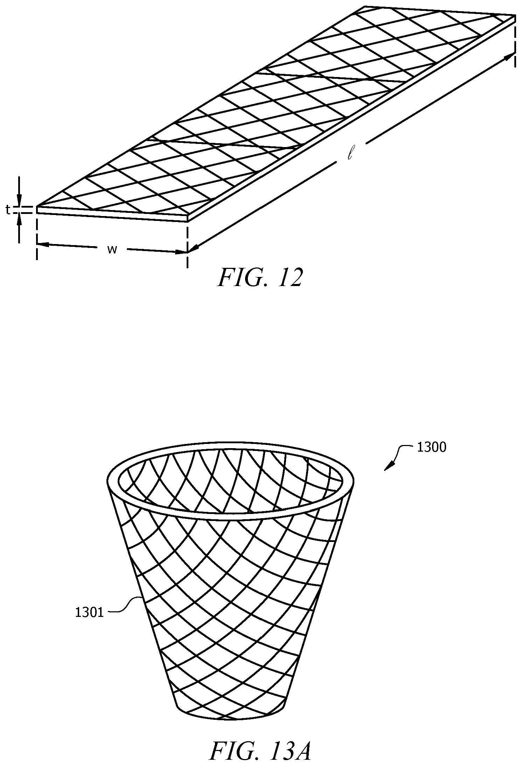

Referring to Figure 12, a first embodiment of a BSS/BAIESS comprising a multi-use sheet is illustrated. In the embodiment of Figure 12, the BSS/BAIESS comprising one or more multi-use sheet may have a length (/) in the range of from about 1 cm to about 4 cm, alternatively, from about 3 cm to about 3 cm. Also, in an embodiment the width (w) may be in a range of from about 1 cm to about 4 cm, alternatively, from about 2 cm to about 3 cm. Also, in an embodiment the sheet may have a thickness (t) in the range of from about 1 mm to about 4 mm, alternatively, from about 2 cm to about 3 cm.

Also, in the embodiment of Figure 12, the multi-use sheet is formed from a plurality of operably-coupled filaments, for example, cumulatively forming a web-like or mesh-like material. In an embodiment, the filaments may have an outside diameter of from about 1 mm to about 2 mm, and a length of from about 10 mm to about 50 mm, alternatively, from about 10 mm to about 40 mm, alternatively, from about 10 mm to about 20 mm, alternatively, from about 20 mm to about 30 mm

In an embodiment, referring to Figure 12, two or more multi-use sheets may be bound together, for example, in a linear fashion so as to allow for longer final shapes for deployment. In such an embodiment, the two or more sections may be bound (e.g., vertically, alternatively horizontally) with two or more filaments, for example, being spaced by about 1 mm apart, thereby allowing the binding filaments to be trimmed so as to separate two adjacent sheets without the possibility of the ends becoming frayed, which would present a potential source of harm (e.g., trauma) at the intended site of deployment (e.g., at the tissue intended to be treated by the multi-use sheet). In an embodiment, the number of sheets conjoined together may be from 1 to about five, alternatively, two, three, four, five, or more.

In an embodiment, the BAIESS comprising a multi-use sheet may comprise a suitable AI or combination of AIs. As will be disclosed herein, the AI and/or combination of AIs may be at least partially dependent upon the patient and/or the conditions experienced by the patient.

In an embodiment, a method of utilizing a BSS/BAIESS comprising such a multi-use sheet may comprise deploying the multi-use sheet within, about, around, or otherwise substantially proximate to the intended site of deployment, for example, as will be disclosed herein.

For example, in an embodiment a BSS/BAIESS comprising a multi-use sheet may be utilized in the treatment of external auditory canal infections including, but not limited to, otitis externa (commonly known as swimmer's ear). In such an embodiment, a physician may deploy such a multi-use sheet (e.g., a rolled, multi-use sheet for example, exhibiting a radially-outward force) within the auditory canal (e.g., the ear canal). Alternatively, in an embodiment, a similar multi-use sheet may be configured for deployment by the patient themselves and/or by non-physician and/or in a non-clinical setting.

In an embodiment where the multi-use sheet is utilized within the auditory canal, the AI(s) may include, but are not limited a corticosteroid such as beclomethasone, budesonide, ciclesonide, fluticasone furoate, fluticasone propionate, flunisolide, mometasone, or triamcinolone, or an antibiotic such as, but not limited to, mupirocin, ciprofloxacin, ofloxacin, or combinations thereof.

Additionally or alternatively, in an embodiment a multi-use sheet may be utilized in the treatment of stenosis of the external auditory canal, such as may result from trauma or a burn. In such an embodiment, a BSS/BAIESS comprising a multi-use sheet may be deployed within the auditory canal and may exhibit a radially-outward force, for example, so as to maintain patency of the auditory canal. Also, in such an embodiment, the BAIESS may deliver one or more AI's such as a corticosteroid such as beclomethasone, budesonide, ciclesonide, fluticasone furoate, fluticasone propionate, flunisolide, mometasone, or triamcinolone, or an antibiotic such as, but not limited to, mupirocin, ciprofloxacin, ofloxacin, or combinations thereof.

In another embodiment a BSS/BAIESS comprising a multi-use sheet may be utilized within the middle ear and/or Eustachian tube, for example, following middle ear a surgical procedure such as tympanoplasty, tympanomastoidecomy, middle ear exploration, ossicular chain reconstruction, stapedectomy, or the like. In such an embodiment, the BSS/BAIESS could be configured (e.g., trimmed) for placement during or following the surgical procedure, for example, so as to provide support to the middle ear or Eustachian tube and, in an embodiment, deliver AI(s) to site of the procedure, for example, to prevent infection, to promote healing, or combinations thereof. In such an embodiment, the AI(s) may include, but are not limited to, a corticosteroid such as beclomethasone, budesonide, ciclesonide, fluticasone furoate, fluticasone propionate, flunisolide, mometasone, or triamcinolone, growth factors, for example, to promote healing, an antibiotic such as, but not limited to, mupirocin, ciprofloxacin, ofloxacin, or combinations thereof.

In additional or alternative embodiments, a BSS/BAIESS comprising a multi-use sheet may be utilized within a lumen or other cavity or passageway including but not limited to, the lacrimal ducts, pharynx, larynx, esophagus, spinal canal, cervical foramen around exiting nerves, tracheobronchial tree, hepatobiliary tract, pancreatic duct, ureters, urethra, Fallopian tube, uterus, vagina, Vas deferens, vascular anastamoses either in vascular surgery or microvascular free tissue transfer, skin grafts. In such an embodiment, the BSS/BAIESS could be configured (e.g., trimmed) for placement during or following a surgical procedure, for example, to repair such lumen or other space. Also, in such an embodiment, the BSS/BAIESS may be configured to maintain the patency of such lumen (e.g., during the healing process) and, in an embodiment, to deliver one or more AI(s), for example, at or substantially proximate to the site of healing. In an embodiment, the AI(s) may include, but are not limited to, a corticosteroid such as beclomethasone, budesonide, ciclesonide, fluticasone furoate, fluticasone propionate, flunisolide, mometasone, or triamcinolone, growth factors, for example, to promote healing, or an antibiotic such as, but not limited to, mupirocin, ciprofloxacin, ofloxacin, or combinations thereof.

### Intrauterine Device Embodiments

In an embodiment, the BSS/BAIESS comprises, is comprised of, and/or is configured to be effective as an intrauterine device (IUD). In such an embodiment, such a BSS/BAIESS may be configured for placement within and/or at least partially within the uterus, for example, by insertion via the cervix. Referring to Figure 13A, a first embodiment of a BSS/BAIESS comprising an IUD device 1300 is illustrated. In the embodiment of Figure 13A, the BSS/BAIESS may be characterized as similar, for example similar in form and/or structure, to a conventional IUD (e.g., a conventional intrauterine contraceptive device). For example, a BSS/BAIESS comprising an IUD may interfere with endometrial implantation of a fertilized egg.

In the embodiment of Figure 13A, the BSS/BAIESS may generally comprise a hollow, cylindrical (e.g., a tube-like) or cone-shaped component 1301 or, alternatively, a sheet configured to rolled. Such an IUD may be formed from a mesh-like material, for example, comprising a plurality of coupled filaments. In an embodiment, such an IUD may be unrolled to form a basketlike or conical shape, for example, as may be approximately the same shape as the uterus.

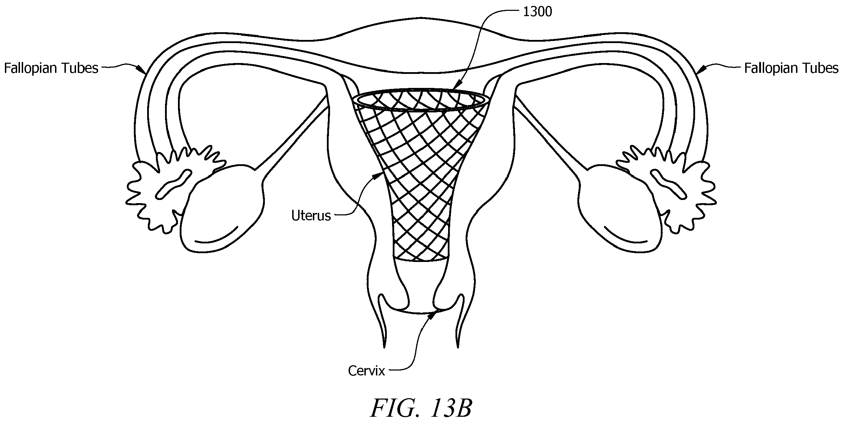

Referring to Figure 13B, an embodiment of a BSS/BAIESS comprising an IUD deployed within the uterus of a patient is illustrated. For example, in such an embodiment, a BSS/BAIESS comprising an IUD may be configured for placement in the uterus and/or Fallopian tube(s) and may be sized or sizeable accordingly, for example, having a diameter and/or a length sized for placement within such structures. In such an embodiment, a BSS/BAIESS comprising an IUD may be deployed within the uterus so as to obscure and/or block at least a portion of the uterine wall.

In an embodiment, a BSS/BAIESS comprising an IUD may be utilized and/or effective as a contraceptive. For example, in such an embodiment, the AI may comprise a hormone, such as estrogen or progesterone or the like, a spermicide, and/or various antibacterial, antiviral, or antifungal agents, for example, to prevent sexually transmitted diseases.

In an alternative embodiment, a BSS/BAIESS comprising an IUD may be utilized in a fertility treatment. For example, in such an embodiment, the AI may comprise anastrozole, clomiphene, metformin, letrozole, progesterone, follitropin, urofolltropin, menotropins, chorionic gonadotropinnafarelin, cetrorelix, ganirelix, lutropin, or the like, pre-natal vitamins, or combinations thereof. In such an embodiment, a BSS/BAIESS comprising an IUD may be configured for placement intravaginally, for example, around the exterior of the cervix, so as to foster a better chemical environment for the semen to penetrate the cervix without irritating the endometrium and allowing for improved potential for pregnancy. In an additional embodiment, a similar device (e.g., similar to the BSS/BAIESS comprising an IUD) may be configured for placement proximate to the uterus (e.g., intravaginally), for example, so as to not interfere with fertilization and/or implantation of a fertilized egg.

In an embodiment, a BSS/BAIESS comprising an IUD may be deployed by a physician. For example, in an embodiment a BSS/BAIESS comprising an IUD may be deployed by utilization of a suitable plunger or syringe-type deployment tool. In an embodiment, the BSS/BAIESS comprising an IUD may be compressible, for example, radially compressible. In such an embodiment, the BSS/BAIESS may be compressed and/or contracted (e.g., radially compressed) loaded into the plunger or syringe-type deployment tool. The deployment tool embodiment may then be brought into the vicinity of the intended site of deployment (e.g., into the uterus and/or Fallopian tube(s)), extruded or otherwise ejected from the deployment device, and allowed to radially expand, for example, thereby allowing the BSS/BAIESS comprising an IUD to contact the uterus and/or Fallopian tube(s).

In an embodiment, a BSS/BAIESS comprising an IUD may be characterized as exhibiting at least about 95% degradation in a duration of from about 6 months to about 12 months, alternatively, from about 8 months to about 10 months, alternatively, about 9 months. In an embodiment, a BAIESS comprising a nasal splint may be characterized as exhibiting at least about 95% elution of the AI in a duration of from about 6 months to about 12 months, alternatively, from about 8 months to about 10 months, alternatively, about 9 months. Additionally, in an embodiment, BSS/BAIESS comprising an IUD may further comprise a contrast media, such as a radiopaque indicator, for example so as to indicate when biodegradation has occurred to an extent so as to necessitate replacement of the IUD.

### Constrictive Device Embodiments

In an embodiment, the BSS/BAIESS comprises, is comprised of, and/or is configured to be effective as a constrictive device. In such an embodiment, the BSS/BAIESS comprising the constrictive device may be configured for placement around a lumen or other passageway, for example, so as to narrow, compress, occlude, constrict, block, reduce flow, or obliterate a potential or actual airway space or fluid channel. In such an embodiment, the BSS/BAIESS comprising the constrictive device may comprise an external collar or clip. For example, in an embodiment, a BSS/BAIESS comprising a constrictive device may be configured to exert a radially-inward (e.g., centripetal), as will be disclosed herein.

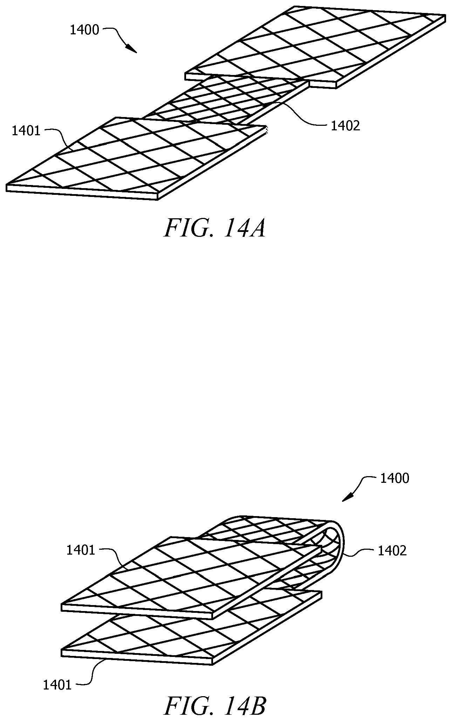

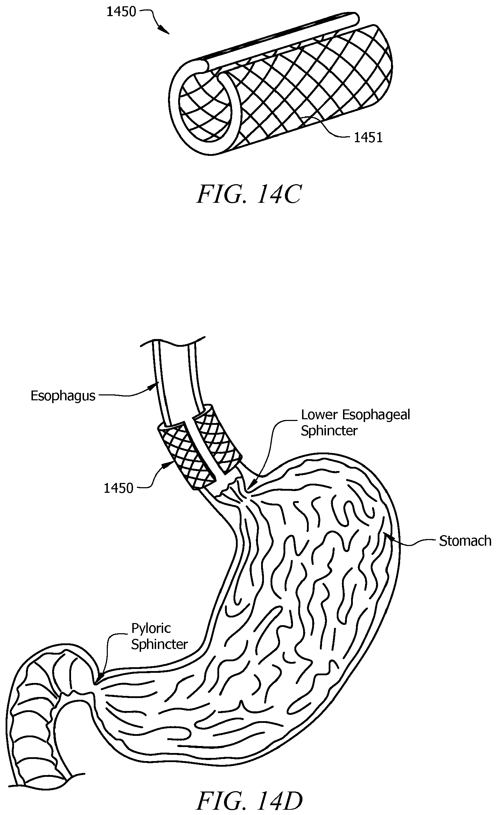

Referring to Figures 14A and 14B, a first embodiment of a BSS/BAIESS comprising a constrictive device is illustrated in an undeployed state and in a deployed state, respectively. In the embodiment of Figures 14A and 14B, the BSS/BAIESS comprising the constrictive device generally comprises a clip 1400. For example, in such an embodiment, such a clip 1400 may comprise two terminal portions 1401 connected by a malleable joint portion 1402 (e.g., a spring-like hinge or joint). In an embodiment, each of the two terminal portions may be formed from a mesh-like material (e.g., a network of intersecting filament, for example, each of the filaments having an outside diameter of from about 1 mm to about 4 mm, and a length of from about 10 mm to about 40 mm, alternatively, from about 10 mm to about 20 mm, alternatively, from about 20 mm to about 30 mm), a film-like material, a sheet-like material, a membrane-like material, or combinations thereof, for example, comprising a degradable polymer. Also, in an embodiment, the malleable joint portion may be formed from one or more thicknesses of a mesh-like material, a film-like material, a sheet-like material, a membrane-like material, or combinations thereof, for example comprising a degradable polymer. Additionally of alternatively, the malleable joint portion may comprise a solid, degradable polymer, and may have a thickness from about 1 mm to about 4 mm, alternatively, from about 1 mm to about 2 mm, alternatively, from about 2 mm to about 3 mm. Additionally, in an embodiment such a BSS/BAIESS comprising a clip may comprise a locking mechanism (e.g., a latch, a tie, or the like), for example, to retain the clip in a closed conformation when deployed. In an embodiment, such a BSS/BAIESS comprising a constrictive device (e.g., a clip) may be deployed by a physician by positioning the clip around or partially around a lumen or other passageway and folding the terminal portions of the clip together via the malleable joint portion, for example, thereby restricting, preventing, inhibiting, or completely occluding flow (e.g., fluid movement) via the lumen or passageway.

Referring to Figure 14C, a second embodiment of a BSS/BAIESS comprising a constriction device is illustrated. In the embodiment of Figure 14C, the BSS/BAIESS comprising a constriction device generally comprises a sleeve or collar 1450. For example, in such an embodiment, such a collar may comprise a sheet 1451 formed from a mesh-like material, a film-like material, a sheet-like material, a membrane-like material, or combinations thereof. Also, in such an embodiment, the sheet may be configured to exhibit a tendency to roll, for example, so as to exhibit a radially inward force (e.g., a centripetal force). For example, the sheet may form an incomplete cylinder, alternatively, an overlapping, constrictive cylinder. In an embodiment, the sheet (e.g., forming the incomplete cylinder) may comprise a gap or slit (for example, being vertically oriented) of about 1 mm to about 5 mm across, alternatively, about 2 mm 4 mm across. For example, the gap or slit would allow the BSS/BAIESS comprising the constrictive device to be wrapped around a lumen, passageway, or other anatomical structure (e.g., an irregularly-shaped structure). In such an embodiment, the BSS/BAIESS comprising the constrictive device, because of the tendency to roll, would then exert a radially-inward, centripetal force, for example, so as to narrow, partially occlude, substantially completely occlude, or completely occlude (e.g., block flow through) the lumen or passageway about which the BSS/BAIESS comprising the constrictive device (e.g., the collar) is deployed.

In an embodiment, a BSS/BAIESS comprising a constrictive device may be utilized in the treatment gastroesophageal reflux disease. In such an embodiment, a BSS/BAIESS comprising a constrictive device may be deployed by a physician in the vicinity of the lower esophageal sphincter, for example, to reduce gastroesophageal reflux disease by narrowing or partially occluding a lower portion of the esophagus. For example, referring to Figure 14B, an embodiment of a BSS/BAIESS comprising a constrictive device is illustrated deployed about and/or around In such an embodiment, the AI could be delivered in the vicinity of the lower esophagus and proximal to the stomach. In an embodiment, the AI(s) may include, but are not limited to, proton-pump inhibitors such as dexlansoprazole, esomeprazole, lansoprazole, omeprazole, pantoprazole, rabeprazole, or combinations thereof, suitable H₂-receptor antagonists such as cimetidine, famotidine, lafutidine, nizatidine, ranitidine, and roxatidine, or the like. As such, not intending to be bound by theory, such a two-pronged method of treatment may improve the condition of patients suffering from gastroesophageal reflux disease and or laryngopharyngeal reflux disease by narrowing the esophageal lumen at its insertion into the stomach and by pharmacologically reducing hydrochloric acid production in the stomach itself, for example, improving the pH (e.g., making the pH closer to a neutral pH) of the stomach contents that could be refluxed into the esophagus and beyond.

In an embodiment, a BSS/BAIESS comprising a constrictive device may be utilized in the treatment of morbid obesity. In such an embodiment, the BSS/BAIESS comprising a constrictive device may be deployed by a physician in the vicinity of the stomach, for example, to reduce to capacity of the stomach for food intake. In such an embodiment, the BSS/BAIESS comprising a constrictive device may be characterized as exhibiting at least about 95% degradation in a duration of from about 1 year to about 3 years, alternatively, about 2 years. In an embodiment, when an AI is present, the AI(s) may include, but are not limited to, proton-pump inhibitors such as dexlansoprazole, esomeprazole, lansoprazole, omeprazole, pantoprazole, rabeprazole, or combinations thereof, suitable H₂-receptor antagonists such as cimetidine, famotidine, lafutidine, nizatidine, ranitidine, and roxatidine, or the like. As such, not intending to be bound by theory, such a BSS/BAIESS comprising a constrictive device may provide constriction of the stomach and, when such an AI is present, preempt to the need for oral administration of H₂-receptor antagonists and/or proton-pump inhibitors.

In another embodiment, a BSS/BAIESS comprising a constrictive device may be utilized in the prevention of vesicoureteral reflux. In such an embodiment, a BSS/BAIESS comprising a constrictive device may be deployed by a physician at the insertion of the ureters into the bladder, for example, to prevent vesicoureteral reflux by narrowing or partially occluding the ureters. For example, a physician could deploy such a constrictive device around the urethral sphincter to improve urinary continence.

In still another embodiment, a BSS/BAIESS comprising a constrictive device may be utilized in the treatment of an arteriovenous malformation or a vascular aneurysm. In such an embodiment, a BSS/BAIESS comprising a constrictive device may be deployed by a physician at the inflow vessel of an arteriovenous malformation or a vascular aneurysm, for example, to substantially or completely occlude vascular flow to such arteriovenous malformation or vascular aneurysm. In such an embodiment, the AI may comprise a vaso-occlusive or sclerosing agents, for example, so as to further inhibit or bring about the complete cessation of vascular flow. Not intending to be bound by theory, utilization of such a BAIESS comprising a constrictive device in the treatment of a arteriovenous malformation or vascular aneurysm may result in the formation of a blockage (e.g., scarring and/or other sclerosis) within vascular tissues flowing into such arteriovenous malformation or vascular aneurysm, for example, resulting in the effective isolation of such arteriovenous malformation or vascular aneurysm. Additionally, after such a blockage has formed, the constrictive device may be allowed to degrade/biodegrade with the expectation that such blockage will remain.

In still another embodiment, a BSS/BAIESS comprising a constrictive device may be utilized in the treatment of a tumor or a region of metastasis. In an additional or alternative embodiment, the AI comprises a chemotherapeutic/ antineoplastic agent, for example, as disclosed herein above. In such an embodiment, such a BSS/BAIESS could be deployed around, about, encompassing, or otherwise proximate to a vascular inflow (e.g., a primary or major inflow) to a tumor or to a region of metastases, and, additionally or alternatively, around, about, encompassing, or otherwise proximate to a vascular outflow or lymphatic outflow, for example, to regionally control the disease and/or inhibit, or prevent metastatic dissemination. Not intending to be bound by theory, utilization of such a BSS/BAIESS comprising a constrictive device in the treatment of a tumor or a region of metastasis may result in the formation of a blockage (e.g., scarring and/or other sclerosis) within vascular and/or lymphatic tissues flow into and/or out of such a tumor or metastatic region, for example, resulting in the effective isolation of such tumor or metastatic region so as to control the progression of, cease the growth of, and/or inhibit metastasis by such tumor or region. Additionally, after such a blockage has formed, the constrictive device may be allowed to degrade/biodegrade with the expectation that such blockage will remain.

In yet another embodiment, a BSS/BAIESS comprising a constrictive device may be utilized in a male or female reproductive sterilization procedure. For example, in such an embodiment, a BSS/BAIESS comprising a constrictive device may be deployed by a physician around, about, encompassing, or otherwise proximate to the Fallopian tubes (e.g., in the case of female reproductive sterilization) or the vas deferens (e.g., in the case of a vasectomy for male reproductive sterilization). In such an embodiment or embodiments, the AI may comprise one or more hormones and/or sclerosing agents, for example, to further inhibit reproductive function. Not intending to be bound by theory, utilization of such a BSS/BAIESS comprising a constrictive device in either a male or female reproductive sterilization procedure may result in the formation of a blockage (e.g., scarring and/or other sclerosis) within an anatomical reproductive structure (e.g., the vas deferens or the Fallopian tubes), for example, resulting in the inhibition of reproductive function by such structure. Additionally, after such a blockage has formed, the constrictive device may be allowed to degrade/biodegrade with the expectation that such blockage will remain.

In an embodiment, a BSS/BAIESS, for example, as disclosed herein, may be considered as introducing revolutionary philosophical and technical changes in therapeutic delivery techniques. Such therapeutic delivery techniques can be employed by healthcare professionals across many different medical specialties, and in some instances, these techniques and devices may even be deployed safely and beneficially by the patients themselves without need for a visit to a healthcare provider.

In an embodiment, the platform (e.g., the support structure of the BSS/BAIESS, as disclosed herein) may be made available in or otherwise tailored to varying shapes, sizes, and configurations, example of which have been disclosed herein, for example, as may be at least partially dependent upon the intended use of the BSS/BAIESS (e.g., the intended or target site of deployment). As disclosed herein, the BSS/BAIESS, particularly, the platform is biodegradable via the body's normal breakdown functions, and can be provided with (e.g., externally or internally) a wide variety of active ingredients. Because the duration associated with biodegradation can be manipulated from a very short period of time (e.g., on the order of a few days) up to a long period of time a period of time (e.g., on the order of several years), the duration that the platform and/or the BSS/BAIESS remains in the body can be tailored to meet specific treatment regimes. Additionally, and similarly, because the duration associated with elution of the active ingredient(s) (for example, which may be attached to, affixed to, or contained within the platform of the BSS/BAIESS), the BSS/BAIESS can also be manipulated so as to maximize and/or optimize the release of the active ingredients for the benefit of the patient.

In various embodiments, the possibilities for treatment of undesired states that may be treated utilizing the BSS/BAIESS and/or the methods of utilizing the same, as disclosed herein, are numerous, and are not confined or in any way limited to the examples disclosed herein. As disclosed herein, the BSS/BAIESS and/or the methods of utilizing the same may be beneficially employed in the treatment of allergic and/or infectious inflammation of the "upper airway" (as used herein, referring to, *inter alia,* the middle ear cavities, the Eustachian tubes, the nasopharynx, the paranasal sinuses, the nasal cavities, the oral cavity, the oropharynx, the hypopharynx, and the larynx). As will be appreciated by one of skill in the art upon viewing this disclosure, such allergic and/or infectious inflammation of the upper airway includes a widespread catalog of diseases, disorder, or other undesired states affecting individual patients so frequently as to have a major economic impact on work and school performance, employer's productivity, and healthcare costs.

In an embodiment, utilization of a BSS/BAIESS, for example, as disclosed herein, in the treatment of such allergic and/or infectious inflammation of the upper airway may result in improved overall treatment regimes, for example, by simultaneously and/or substantially improving patency of a patient's airway and delivering (e.g., targeting) active ingredients at or proximate to the site of such infectious or inflammatory upper airway disease. Any such improvement in treatment regimens (e.g., improvements in therapeutic drug delivery and/or improvement in the patency of a patient's airway) could have potentially enormous positive economic or educational impact for patients, employers, and/or healthcare insurers. Additionally, improving control of an infectious disease (for example, within a given geographic area), via utilization of efficacious BSS/BAIESS therapies, as disclosed herein, may have a beneficial impact from a public health standpoint, for example, by minimizing the risk of epidemics and pandemics, such as the annual risks associated with influenza and parainfluenza viruses.

In another embodiment, utilization of a BSS/BAIESS, for example, as disclosed herein, in the treatment of a collapse, for example of a transient airway (for example, as may be associated with obstructive sleep apnea or snoring) and/or in conjunction with one or more procedures employed to treat such undesirable states, may be advantageous by improving the speed and degree of healing, minimizing adverse events, minimizing scarring at the site of such procedure (e.g., an operative site), or combinations thereof. Often, such scarring may limit the beneficial effects of the original corrective procedure to the patient, and may even lead the necessity of secondary, revision procedures having associated deleterious medical, psychological, and economic impacts.

In another embodiment, utilization of a BSS/BAIESS, for example, as disclosed herein, in the treatment of an undesirable state and/or a procedure associated with the lower airway (as used herein, referring to, *inter alia,* the tracheobroncial tree and the lungs themselves), the ocular drainage system (nasolacrimal ducts), the gastrointestinal tract (e.g., extending from the esophagus down to the rectum and anus), the pancreatic-hepatobiliary system, the various tubular components of the genitourinary tract (including, *inter alia,* the ureters, bladder, urethra, Fallopian tubes, uterus, vagina, and vas deferens, the vertebral and spinal nerve entrance/exit canals), the arteriovenous network of blood vessels may be advantageous by providing for and/or improving the targeted delivery of an active ingredient to a desired tissue. Similarly, a BSS/BAIESS may be advantageously employed in medical specialties charged with reconstructing tissue defects, for example, by skin grafting, microvascular free tissue transfer, or the like. Not intending to be bound by theory, a BSS/BAIESS, as disclosed herein, has the capacity to improve the result of a patient's treatment regime by increasing local delivery of one or more active ingredients, while minimizing or eliminating completely unwanted systemic side effects and/or by at least partially commensurately, providing structural support to a tissue or tissues during the healing process. As will be appreciated by one of skill in the art upon viewing this disclosure, the BSS/BAIESS may provide an avenue by which to achieve one of the ultimate pursuits of the artists and scientists who research and practice medicine: the development of a treatment regime allowing for targeted, effective delivery of an active ingredient within the body substantially proximate to, if not precisely, where it is needed, thereby minimizing the negative impacts associated with delivery of such an active ingredient to places in the body where it is not needed.

Additionally, respiratory diseases (such as those discussed herein resulting from the influenza viruses) are not the only causes of epidemics and pandemics. In another embodiment, a BSS/BAIESS may be utilized to treat or even prevent common sexually-transmitted diseases, even in poorly-compliant patients who are unable or unwilling to seek medical care for the prevention of the propagation of sexually-transmitted diseases.

Further, in an embodiment, a BSS/BAIESS may be utilized in the care of the female of child-bearing age. In an embodiment, a BSS/BAIESS utilized as a fertility treatment, for example, as disclosed herein, may pose multiple advantages in comparison to conventional fertility treatments. For example, a BSS/BAIESS utilized as a fertility treatment may provide both structural support, for example to maintain patency of tubular structures that could be too narrow to allow conception, while simultaneously delivering therapeutic agents to promote ovulation and/or to optimize the local environment, allowing for improved potential for fertilization and implantation.

Similarly, in an embodiment, a BSS/BAIESS may be utilized in a male infertility treatment. In an embodiment, a BSS/BAIESS may be advantageously employed in such a male infertility treatment, for example, by promoting patency, proper anastamotic healing, and sperm production following a vasectomy reversal or repair of an occlusion to the vas deferens.

Further still, a BSS/BAIESS may be advantageously employed in contraception. For example, as disclosed herein, a BSS/BAIESS may be employed to prevent unintentional, unwanted pregnancies by being placed intravaginally, peri-cervically, or within the uterus itself, for example, comprising active hormones, spermicides, antiviral, and/or antibiotic ingredients, while simultaneously serving as a barriers capable of physically blocking pathogen invasion or egg implantation. As such, for example, the utilization of a BSS/BAIESS in contraception may be advantageous, in comparison to conventional means of contraception, in that a BSS/BAIESS so-utilized may simultaneously provide multiple means (e.g., both via a physical barrier and via the delivery of an active ingredient, such as a hormone or spermicide) to prevent unintended pregnancy.

Further still, a BSS/BAIESS may be advantageously employed in oncology. In an embodiment, a BSS/BAIESS utilized in oncology, for example, to treat a malignant tumor or a metastatic region, may be advantageous in that a BSS/BAIESS could be placed around a malignancy itself, or within the vascular supply to the tumor or area of metastases, to deliver a chemotherapeutic substantially proximate to, if not precisely, where it is needed, thereby minimizing downstream side effects. As noted herein, while all medical interventions require a risk-benefit analysis, such an analysis is a critical factor in the evaluation and management of a cancer patient. Even a slight reduction in the side effects associated with a chemotherapeutic agent, for example as may be provided by utilization of a BSS/BAIESS as disclosed herein, could have major, positive quality-of-life and overall prognosis implications for a cancer patient.

One of skill in the art, upon viewing this disclosure, will appreciate one or more additional embodiments and/or variations of a BSS/BAIESS as disclosed herein and/or methods of utilizing the same. As such, the forgoing is in no way intended to be limited to the embodiments or example disclosed herein.

### ADDITIONAL EMBODIMENTS

The following are nonlimiting, specific embodiments in accordance with the present disclosure:

A first embodiment, which is biodegradable nasal splint, wherein the biodegradable nasal splint is configured for placement within a nasal passage so as to stabilize a surgically-corrected septum

A second embodiment, which is the biodegradable nasal splint of the first embodiment, wherein the biodegradable splint is configured to exert a radially outward force.

A third embodiment, which is the biodegradable nasal splint of one of the first through the second embodiments, wherein the biodegradable splint comprises at least 90% by weight of one or more degradable materials.

A fourth embodiment, which is the biodegradable nasal splint of the fourth embodiment, wherein the degradable material is configured such that at least 95% by weight of the degradable material will degrade in a duration of from about 7 days to about 30 days upon deployment within a nasal passage.

A fifth embodiment, which is the biodegradable nasal splint of one of the first through the fourth embodiments, further comprising an active ingredient comprising a corticosteroid, an antihistamine, an anticholinergic agent, an antibiotic, or combinations thereof.

A sixth embodiment, which is the biodegradable nasal splint of the fifth embodiment, wherein the corticosteroid comprises beclomethasone, budesonide, ciclesonide, fluticasone furoate, fluticasone propionate, flunisolide, mometasone, triamcinolone, or combinations thereof.

A seventh embodiment, which is the biodegradable nasal splint of one of the fifth through the sixth embodiments, wherein the antihistamine comprises azelastine, olopatadine, or combinations thereof.

An eighth embodiment, which is the biodegradable nasal splint of one of the fifth through the seventh embodiments, wherein the anticholinergic agent comprises ipratroprium bromide.

A ninth embodiment, which is the biodegradable nasal splint of one of the fifth through the eighth embodiments, wherein the antibiotic comprises mupirocin, doxycycline, trimethoprim/sulfamethoxasole, levofloxacin, or combinations thereof.

A tenth embodiment, which is biodegradable nasal splint, wherein the biodegradable nasal splint is configured for placement within a nasal passage so as to exert a lateralizing force to an outfractured inferior turbinate.

An eleventh embodiment, which is the biodegradable nasal splint of the tenth embodiment, wherein the biodegradable splint is configured to exert a radially outward force.

A twelfth embodiment, which is the biodegradable nasal splint of one of the tenth through the eleventh embodiment, wherein the biodegradable splint comprises at least 90% by weight of one or more degradable materials.

A thirteenth embodiment, which is the biodegradable nasal splint of the twelfth embodiment, wherein the degradable material is configured such that at least 95% by weight of the degradable material will degrade in a duration of from about 7 days to about 30 days upon deployment within a nasal passage.

A fourteenth embodiment, which is the biodegradable nasal splint of one of the tenth through the thirteenth embodiments, further comprising an active ingredient comprising a corticosteroid, an antihistamine, an anticholinergic agent, an antibiotic, or combinations thereof.

A fifteenth embodiment, which is the biodegradable nasal splint of one of the tenth through the fourteenth embodiments, wherein the corticosteroid comprises beclomethasone, budesonide, ciclesonide, fluticasone furoate, fluticasone propionate, flunisolide, mometasone, triamcinolone, or combinations thereof.

A sixteenth embodiment, which is the biodegradable nasal splint of one of the fourteenth through the fifteenth embodiments, wherein the antihistamine comprises azelastine, olopatadine, or combinations thereof.

A seventeenth embodiment, which is the biodegradable nasal splint of one of the fourteenth through the sixteenth embodiments, wherein the anticholinergic agent comprises ipratroprium bromide.

An eighteenth embodiment, which is the biodegradable nasal splint of one of the fourteenth through the seventeenth embodiments, wherein the antibiotic comprises mupirocin, doxycycline, trimethoprim/sulfamethoxasole, levofloxacin, or combinations thereof.

A nineteenth embodiment, which is a biodegradable nasal splint, wherein the biodegradable nasal splint is deployed within a nasal cavity between the nasal septum and an inferior turbinate.

A twentieth embodiment, which is the biodegradable nasal splint of nineteenth embodiment, wherein the biodegradable nasal splint is configured to exert a radially outward force.

A twenty-first embodiment, which is the biodegradable nasal splint of one of the nineteenth through the twentieth embodiments, wherein the biodegradable nasal splint is configured to stabilize a surgically-corrected nasal septum, to exert a lateralizing force to the inferior turbinate, or combinations thereof.

A twenty-second embodiment, which is the biodegradable nasal splint of one of the nineteenth through the twenty-first embodiments, wherein the biodegradable splint comprises at least 90% by weight of one or more degradable materials.

A twenty-third embodiment, which is the biodegradable nasal splint of one of the nineteenth through the twenty-second embodiments, further comprising an active ingredient comprising a corticosteroid, an antihistamine, an anticholinergic agent, an antibiotic, or combinations thereof.

A twenty-fourth embodiment, which is the biodegradable nasal splint of the twenty-third embodiment, wherein the corticosteroid comprises beclomethasone, budesonide, ciclesonide, fluticasone furoate, fluticasone propionate, flunisolide, mometasone, triamcinolone, or combinations thereof.

A twenty-fifth embodiment, which is the biodegradable nasal splint of one of the twenty-third through the twenty-fourth embodiments, wherein the antihistamine comprises azelastine, olopatadine, or combinations thereof.

A twenty-sixth embodiment, which is the biodegradable nasal splint of one of the twenty-third through the twenty-fifth embodiments, wherein the anticholinergic agent comprises ipratroprium bromide.

A twenty-seventh embodiment, which is the biodegradable nasal splint of one of the twenty-third through the twenty-sixth embodiments, wherein the antibiotic comprises mupirocin, doxycycline, trimethoprim/sulfamethoxasole, levofloxacin, or combinations thereof.

A twenty-eighth embodiment, which is a method comprising performing a corrective procedure with respect to a patient's nasal passage; and positioning a biodegradable nasal splint within the patient's nasal passage, wherein the biodegradable nasal splint is effective to exert a lateralizing force to an inferior turbinate, to stabilize a nasal septum, or combinations thereof.

A twenty-ninth embodiment, which is the method of the twenty-eighth embodiment, wherein performing the corrective procedure with respect to the patient's nasal passage comprises medializing the nasal septum.

A thirtieth embodiment, which is the method of one of the twenty-eight through the twenty-ninth embodiments, wherein performing the corrective procedure with respect to the patient's nasal passage comprises lateralizing the inferior turbinate.

A thirty-first embodiment, which is the method of one of the twenty-eight through the thirtieth embodiments, further comprising allowing at least a portion of the biodegradable nasal splint to degrade within the patient's nasal passage.

A thirty-second embodiment, which is the method of one of the twenty-eight through the thirty-first embodiments, wherein the patient is characterized as experiencing allergic rhinitis, viral rhinitis, rhinosinusitis of a viral, a bacterial, or unknown origin, vasomotor rhinitis, or combinations thereof.

A thirty-third embodiment, which is the method of one of the twenty-eight through the thirty-second embodiments, wherein the patient is characterized as experiencing an external and/or internal nasal valve collapse.

A thirty-fourth embodiment, which is the method of one of the twenty-eight through the thirty-third embodiments, wherein the patient is characterized as experiencing snoring and/or obstructive sleep apnea.

A thirty-fifth embodiment, which is the method of one of the twenty-eight through the thirty-fourth embodiments, wherein the patient is characterized as having recently undergone a septoplasty, an inferior turbinate reduction, or combinations thereof

A thirty-sixth embodiment, which is the method of one of the twenty-eight through the thirty-fifth embodiments, wherein the biodegradable splint is configured to exert a radially outward force.

A thirty-seventh embodiment, which is the method of one of the twenty-eight through the thirty-sixth embodiments, wherein the biodegradable splint comprises at least 90% by weight of one or more degradable materials.

A thirty-eighth embodiment, which is the method of thirty-seventh embodiment, wherein the degradable material is configured such that at least 95% by weight of the degradable material will degrade in a duration of from about 7 days to about 30 days upon deployment within a nasal passage.

A thirty-ninth embodiment, which is the method of one of the twenty-eight through the thirty-eighth embodiments, wherein the biodegradable nasal splint further comprises an active ingredient comprising a corticosteroid, an antihistamine, an anticholinergic agent, an antibiotic, or combinations thereof.

A fortieth embodiment, which is the method of the thirty-ninth embodiment, wherein the corticosteroid comprises beclomethasone, budesonide, ciclesonide, fluticasone furoate, fluticasone propionate, flunisolide, mometasone, triamcinolone, or combinations thereof.

A forty-first embodiment, which is the method of one of the thirty-ninth through the fortieth embodiments, wherein the antihistamine comprises azelastine, olopatadine, or combinations thereof.

A forty-second embodiment, which is the method of one of the thirty-ninth through the forty-first embodiments, wherein the anticholinergic agent comprises ipratroprium bromide.

A forty-third embodiment, which is the method of one of the thirty-ninth through the forty-first embodiments, wherein the antibiotic comprises mupirocin, doxycycline, trimethoprim/sulfamethoxasole, levofloxacin, or combinations thereof.

A forty-fourth embodiment, which is a method comprising providing a biodegradable nasal splint, wherein the biodegradable nasal splint is configured for placement within a nasal passage so as stabilize a surgically-corrected septum, to exert a lateralizing force to an outfractured inferior turbinate, or combinations thereof; and packaging the biodegradable nasal splint.

A forty-fifth embodiment, which is the method of the forty-fourth embodiment, further comprising providing instructions for positioning the biodegradable nasal splint within a patient's nasal passage.

A forty-sixth embodiment, which is the method of one of the forty-fourth through the forty-fifth embodiments, further comprising distributing the biodegradable nasal splint for use by a physician.

A forty-seventh embodiment, which is the method of one of the forty-fourth through the forty-sixth embodiments, further comprising distributing the biodegradable nasal splint for over-the-counter distribution to an end-user.

A forty-eighth embodiment, which is the method of one of the forty-fourth through the forty-seventh embodiments, wherein the biodegradable splint is configured to exert a radially outward force.

A forty-ninth embodiment, which is the method of one of the forty-fourth through the forty-eighth embodiments, wherein the biodegradable splint comprises at least 90% by weight of one more degradable materials.

A fiftieth embodiment, which is the method of the forty-ninth embodiment, wherein the degradable material is configured such that at least 95% by weight of the degradable material will degrade in a duration of from about 7 days to about 30 days upon deployment within a nasal passage.

A fifty-first embodiment, which is the method of one of the forty-fourth through the fiftieth embodiments, wherein the biodegradable nasal splint further comprises an active ingredient comprising a corticosteroid, an antihistamine, an anticholinergic agent, an antibiotic, or combinations thereof.

A fifty-second embodiment, which is the method of the fifty-first embodiment, wherein the corticosteroid comprises beclomethasone, budesonide, ciclesonide, fluticasone furoate, fluticasone propionate, flunisolide, mometasone, triamcinolone, or combinations thereof.

A fifty-third embodiment, which is the method of one of the fifty-first through the fifty-second embodiments, wherein the antihistamine comprises azelastine, olopatadine, or combinations thereof.

A fifty-fourth embodiment, which is the method of one of the fifty-first through the fifty-third embodiments, wherein the anticholinergic agent comprises ipratroprium bromide.

A fifty-fifth embodiment, which is the method of one of the fifty-first through the fifty-fourth embodiments, wherein the antibiotic comprises mupirocin, doxycycline, trimethoprim/sulfamethoxasole, levofloxacin, or combinations thereof.

A fifty-sixth embodiment, which is a method comprising opening a package comprising a biodegradable nasal splint, wherein the biodegradable nasal splint is configured for placement within a nasal passage so as stabilize a surgically-corrected septum, to exert a lateralizing force to an outfractured inferior turbinate, or combinations thereof; and allowing at least a portion of the biodegradable nasal splint to degrade within the patient's nasal passage, wherein the biodegradable nasal splint has been previously positioned with the patient's nasal passage.

A fifty-seventh embodiment, which is the method of the fifty-sixth embodiment, wherein the biodegradable splint is configured to exert a radially outward force.

A fifty-eight embodiment, which is the method of one of the fifty-sixth through the fifty-seventh embodiments, wherein the biodegradable splint comprises at least 90% by weight of one or more degradable materials.

A fifty-ninth embodiment, which is the method of the fifty-eighth embodiment, wherein the degradable material is configured such that at least 95% by weight of the degradable material will degrade in a duration of from about 7 days to about 30 days upon deployment within a nasal passage.

A sixtieth embodiment, which is the method of one of the fifty-sixth through the fifty-ninth embodiments, wherein the biodegradable nasal splint further comprises an active ingredient comprising a corticosteroid, an antihistamine, an anticholinergic agent, an antibiotic, or combinations thereof.

A sixty-first embodiment, which is the method of the sixtieth embodiment, wherein the corticosteroid comprises beclomethasone, budesonide, ciclesonide, fluticasone furoate, fluticasone propionate, flunisolide, mometasone, triamcinolone, or combinations thereof.

A sixty-second embodiment, which is the method of one of the sixtieth through the sixty-first embodiments, wherein the antihistamine comprises azelastine, olopatadine, or combinations thereof.

A sixty-third embodiment, which is the method of one of the sixtieth through the sixty-second embodiments, wherein the anticholinergic agent comprises ipratroprium bromide.

A sixty-fourth embodiment, which is the method of one of the sixtieth through the sixty-third embodiments, wherein the antibiotic comprises mupirocin, doxycycline, trimethoprim/sulfamethoxasole, levofloxacin, or combinations thereof.

A sixty-fifth embodiment, which is a method comprising positioning a biodegradable nasal splint within a nasal passage of a patient in need thereof, wherein the biodegradable nasal splint is effective to exert a lateralizing force to an inferior turbinate, to stabilize a nasal septum, or combinations thereof.

A sixty-sixth embodiment, which is the method of the sixty-fifth embodiment, further comprising allowing at least a portion of the biodegradable nasal splint to degrade within the patient's nasal passage.

A sixty-seventh embodiment, which is the method of one of the sixty-fifth through the sixty-sixth embodiments, wherein the patient is characterized as experiencing allergic rhinitis, viral rhinitis, rhinosinusitis of a viral, a bacterial, or unknown origin, vasomotor rhinitis, or combinations thereof.

A sixty-eighth embodiment, which is the method of one of the sixty-fifth through the sixty-seventh embodiments, wherein the patient is characterized as experiencing an external and/or internal nasal valve collapse.

A sixty-ninth embodiment, which is the method of one of the sixty-fifth through the sixty-eighth embodiments, wherein the patient is characterized as experiencing snoring and/or obstructive sleep apnea.

A seventieth embodiment, which is the method of one of the sixty-fifth through the sixty-ninth embodiments, wherein the patient is characterized as having recently undergone a septoplasty, an inferior turbinate reduction, or combinations thereof

A seventy-first embodiment, which is the method of one of the sixty-fifth through the seventieth embodiments, wherein the biodegradable splint is configured to exert a radially outward force.

A seventy-second embodiment, which is the method of one of the sixty-fifth through the seventy-first embodiments, wherein the biodegradable splint comprises at least 90% by weight of one or more degradable materials.

A seventy-third embodiment, which is the method of the seventy-second embodiment, wherein the degradable material is configured such that at least 95% by weight of the degradable material will degrade in a duration of from about 7 days to about 30 days upon deployment within a nasal passage.

A seventy-fourth embodiment, which is the method of one of the sixty-fifth through the seventy-third embodiments, wherein the biodegradable nasal splint further comprises an active ingredient comprising a corticosteroid, an antihistamine, an anticholinergic agent, an antibiotic, or combinations thereof.

A seventy-fifth embodiment, which is the method of the seventy-fourth through embodiment, wherein the corticosteroid comprises beclomethasone, budesonide, ciclesonide, fluticasone furoate, fluticasone propionate, flunisolide, mometasone, triamcinolone, or combinations thereof.

A seventy-sixth embodiment, which is the method of one of the seventy-fourth through the seventy-fifth embodiments, wherein the antihistamine comprises azelastine, olopatadine, or combinations thereof.

A seventy-seventh embodiment, which is the method of one of the seventy-fourth through the seventy-sixth embodiments, wherein the anticholinergic agent comprises ipratroprium bromide.

A seventy-eighth embodiment, which is the method of one of the seventy-fourth through the seventy-seventh embodiments, wherein the antibiotic comprises mupirocin, doxycycline, trimethoprim/sulfamethoxasole, levofloxacin, or combinations thereof.

A seventy-ninth embodiment, which is the use of a biodegradable nasal splint, wherein the biodegradable nasal splint is effective to exert a lateralizing force to an inferior turbinate, to stabilize a nasal septum, or combinations thereof, for the treatment of a patient in need thereof.

An eightieth embodiment, which is the use of the seventy-ninth embodiment, wherein the patient is characterized as experiencing allergic rhinitis, viral rhinitis, rhinosinusitis of a viral, a bacterial, or unknown origin, vasomotor rhinitis, or combinations thereof.

An eighty-first embodiment, which is the use of one of the seventy-ninth through the eightieth embodiments, wherein the patient is characterized as experiencing an external and/or internal nasal valve collapse.

An eighty-second embodiment, which is the use of one of the seventy-ninth through the eighty-first embodiments, wherein the patient is characterized as experiencing snoring and/or obstructive sleep apnea.

An eighty-third embodiment, which is the use of one of the seventy-ninth through the eighty-second embodiments, wherein the patient is characterized as having recently undergone a septoplasty, an inferior turbinate reduction, or combinations thereof

An eighty-fourth embodiment, which is the use of one of the seventy-ninth through the eighty-third embodiments, wherein the biodegradable splint is configured to exert a radially outward force.

An eighty-fifth embodiment, which is the use of one of the seventy-ninth through the eighty-fourth embodiments, wherein the biodegradable splint comprises at least 90% by weight of one or more degradable materials.

An eighty-sixth embodiment, which is the use of the eighty-first embodiment, wherein the degradable material is configured such that at least 95% by weight of the degradable material will degrade in a duration of from about 7 days to about 30 days upon deployment within a nasal passage.

An eighty-seventh embodiment, which is the use of one of the seventy-ninth through the eighty-sixth embodiments, wherein the biodegradable nasal splint further comprises an active ingredient comprising a corticosteroid, an antihistamine, an anticholinergic agent, an antibiotic, or combinations thereof.

An eighty-eighth embodiment, which is the use of the eighty-seventh embodiment, wherein the corticosteroid comprises beclomethasone, budesonide, ciclesonide, fluticasone furoate, fluticasone propionate, flunisolide, mometasone, triamcinolone, or combinations thereof.

An eighty-ninth embodiment, which is the use of one of the eighty-seventh through the eighty-eighth embodiments, wherein the antihistamine comprises azelastine, olopatadine, or combinations thereof.

A ninetieth embodiment, which is use of one of the eighty-seventh through the eighty-ninth embodiments, wherein the anticholinergic agent comprises ipratroprium bromide.

A ninety-first embodiment, which is the use of one of the eighty-seventh through the ninetieth embodiments, wherein the antibiotic comprises mupirocin, doxycycline, trimethoprim/sulfamethoxasole, levofloxacin, or combinations thereof.

At least one embodiment is disclosed and variations, combinations, and/or modifications of the embodiment(s) and/or features of the embodiment(s) made by a person having ordinary skill in the art are within the scope of the disclosure. Alternative embodiments that result from combining, integrating, and/or omitting features of the embodiment(s) are also within the scope of the disclosure. Where numerical ranges or limitations are expressly stated, such express ranges or limitations should be understood to include iterative ranges or limitations of like magnitude falling within the expressly stated ranges or limitations (e.g., from about 1 to about 10 includes, 2, 3, 4, etc.; greater than 0.10 includes 0.11, 0.12, 0.13, etc.). For example, whenever a numerical range with a lower limit, Rl, and an upper limit, Ru, is disclosed, any number falling within the range is specifically disclosed. In particular, the following numbers within the range are specifically disclosed: R=R1+k^{∗}(Ru-R1), wherein k is a variable ranging from 1 percent to 100 percent with a 1 percent increment, i.e., k is 1 percent, 2 percent, 3 percent, 4 percent, 5 percent, ..., 50 percent, 51 percent, 52 percent, ..., 95 percent, 96 percent, 97 percent, 98 percent, 99 percent, or 100 percent. Moreover, any numerical range defined by two R numbers as defined in the above is also specifically disclosed. Use of the term "optionally" with respect to any element of a claim means that the element is required, or alternatively, the element is not required, both alternatives being within the scope of the claim. Use of broader terms such as comprises, includes, and having should be understood to provide support for narrower terms such as consisting of, consisting essentially of, and comprised substantially of. Accordingly, the scope of protection is not limited by the description set out above but is defined by the claims that follow, that scope including all equivalents of the subject matter of the claims. Each and every claim is incorporated as further disclosure into the specification and the claims are embodiment(s) of the present invention.

### CLAUSES

1. A biodegradable nasal splint, wherein the biodegradable nasal splint is configured for placement within a nasal passage so as to stabilize a surgically-corrected septum.
2. The biodegradable nasal splint of clause 1, wherein the biodegradable splint is configured to exert a radially outward force.
3. The biodegradable nasal splint of clause 1, wherein the biodegradable splint comprises at least 90% by weight of one or more degradable materials.
4. The biodegradable nasal splint of clause 4, wherein the degradable material is configured such that at least 95% by weight of the degradable material will degrade in a duration of from about 7 days to about 30 days upon deployment within a nasal passage.
5. The biodegradable nasal splint of clause 1, further comprising an active ingredient comprising a corticosteroid, an antihistamine, an anticholinergic agent, an antibiotic, or combinations thereof.
6. The biodegradable nasal splint of clause 5,
   wherein the corticosteroid comprises beclomethasone, budesonide, ciclesonide, fluticasone furoate, fluticasone propionate, flunisolide, mometasone, triamcinolone, or combinations thereof,
   wherein the antihistamine comprises azelastine, olopatadine, or combinations thereof,
   wherein the anticholinergic agent comprises ipratroprium bromide,
   wherein the antibiotic comprises mupirocin, doxycycline, trimethoprim/sulfamethoxasole, levofloxacin, or combinations thereof, or
   combinations thereof.
7. A biodegradable nasal splint, wherein the biodegradable nasal splint is configured for placement within a nasal passage so as to exert a lateralizing force to an outfractured inferior turbinate.
8. The biodegradable nasal splint of clause 7, wherein the biodegradable splint is configured to exert a radially outward force.
9. The biodegradable nasal splint of clause 7, wherein the biodegradable splint comprises at least 90% by weight of one or more degradable materials.
10. The biodegradable nasal splint of clause 9, wherein the degradable material is configured such that at least 95% by weight of the degradable material will degrade in a duration of from about 7 days to about 30 days upon deployment within a nasal passage.
11. The biodegradable nasal splint of clause 7, further comprising an active ingredient comprising a corticosteroid, an antihistamine, an anticholinergic agent, an antibiotic, or combinations thereof.
12. The biodegradable nasal splint of clause 11,
   wherein the corticosteroid comprises beclomethasone, budesonide, ciclesonide, fluticasone furoate, fluticasone propionate, flunisolide, mometasone, triamcinolone, or combinations thereof,
   wherein the antihistamine comprises azelastine, olopatadine, or combinations thereof,
   wherein the anticholinergic agent comprises ipratroprium bromide,
   wherein the antibiotic comprises mupirocin, doxycycline, trimethoprim/sulfamethoxasole, levofloxacin, or combinations thereof, or
   combinations thereof.
13. A method comprising:
   performing a corrective procedure with respect to a patient's nasal passage;
   positioning a biodegradable nasal splint within the patient's nasal passage, wherein the biodegradable nasal splint is effective to exert a lateralizing force to an inferior turbinate, to stabilize a nasal septum, or combinations thereof.
14. The method of clause 13, wherein performing the corrective procedure with respect to the patient's nasal passage comprises medializing the nasal septum.
15. The method of clause 13, wherein performing the corrective procedure with respect to the patient's nasal passage comprises lateralizing the inferior turbinate.
16. The method of clause 13, further comprising allowing at least a portion of the biodegradable nasal splint to degrade within the patient's nasal passage.
17. The method of clause 13, wherein the patient is characterized as experiencing allergic rhinitis, viral rhinitis, rhinosinusitis of a viral, a bacterial, or unknown origin, vasomotor rhinitis, or combinations thereof.
18. The method of clause 13, wherein the patient is characterized as experiencing an external and/or internal nasal valve collapse, snoring and/or obstructive sleep apnea, or combinations thereof.
19. The method of clause 13, wherein the biodegradable splint comprises at least 90% by weight of one or more degradable materials.
20. The method of clause 13, wherein the biodegradable nasal splint further comprises an active ingredient comprising a corticosteroid, an antihistamine, an anticholinergic agent, an antibiotic, or combinations thereof.

## Claims

1. A biodegradable nasal support configured for placement within a paranasal sinus or an opening to the paranasal sinus of a patient, the biodegradable nasal support comprising:
a tubular structure having a tubular wall at least partially defining an interior lumen,
wherein the biodegradable nasal support consists essentially of a degradable material and, optionally, an active ingredient, the degradable material comprising chitosan and a polymer comprising acrylic acid monomers.

2. The biodegradable nasal support of claim 1, wherein the tubular wall is solid.

3. The biodegradable nasal support of claim 1, wherein the tubular wall comprises a webbed platform having a unitary structure and formed by a plurality of strands that collectively define a plurality of open spaces within the tubular wall.

4. The biodegradable nasal support of claim 1, wherein the biodegradable splint comprises at least 95% by weight of the degradable material.

5. The biodegradable nasal support of claim 4, wherein the degradable material is configured such that at least 95% by weight of the degradable material will degrade in a duration of from about 7 days to about 30 days upon deployment within the paranasal sinus or the opening to the paranasal sinus.

6. The biodegradable nasal support of claim 1, wherein the degradable material comprises at least 35% of the chitosan by weight of the degradable material and at least 35% of the polymer comprising acrylic acid monomers by weight of the degradable material.

7. The biodegradable nasal support of claim 6, wherein the degradable material further comprises polyethylene glycol.

8. The biodegradable nasal support of claim 7, wherein the degradable material comprises at least 20% of the polyethylene glycol by weight of the degradable material.

9. The biodegradable nasal support of claim 1, wherein the active ingredient comprises a corticosteroid, an antihistamine, an anticholinergic agent, an antibiotic, an antiviral, an antifungal, or combinations thereof.

10. The biodegradable nasal support of claim 9,
wherein the corticosteroid comprises beclomethasone, budesonide, ciclesonide, fluticasone furoate, fluticasone propionate, flunisolide, mometasone, triamcinolone, or combinations thereof,
wherein the antihistamine comprises azelastine, olopatadine, loratadine, desloratadine, fexofenadine, cetirizine, levocetirizine, or combinations thereof,
wherein the anticholinergic agent comprises ipratroprium bromide, meclozine, or combinations thereof,
wherein the antibiotic comprises mupirocin, doxycycline, trimethoprim/sulfamethoxasole, levofloxacin, ciprofloxacin, cephalexin, amoxicillin, clavulanic acid or combinations thereof,
wherein the antiviral comprises acyclovir, valcyclovir, or combinations thereof, or
wherein the antifungal comprises an allylamine, an imidazole, a polyenes, a thiocarbamate, a triazole, a derivative thereof, or combinations thereof.

11. A method for providing therapy to a patient, the method comprising
disposing a biodegradable nasal support within a paranasal sinus or an opening to the paranasal sinus of the patient, the biodegradable nasal support comprising a tubular structure having a tubular wall at least partially defining an interior lumen, wherein the biodegradable nasal support consists essentially of a degradable material and, optionally, an active ingredient, the degradable material comprising chitosan and a polymer comprising acrylic acid monomers.

12. The method of claim 11, wherein the tubular wall is solid.

13. The method of claim 11, wherein the tubular wall comprises a webbed platform having a unitary structure and formed by a plurality of strands that collectively define a plurality of open spaces within the tubular wall.

14. The method of claim 11, wherein the biodegradable splint comprises at least 95% by weight of the degradable material.

15. The method of claim 14, wherein the degradable material is configured such that at least 95% by weight of the degradable material will degrade in a duration of from about 7 days to about 30 days upon deployment within the paranasal sinus or the opening to the paranasal sinus.

16. The method of claim 11, wherein the degradable material comprises at least 35% of the chitosan by weight of the degradable material and at least 35% of the polymer comprising acrylic acid monomers by weight of the degradable material.

17. The method of claim 16, wherein the degradable material further comprises polyethylene glycol.

18. The method of claim 17, wherein the degradable material comprises at least 20% of the polyethylene glycol by weight of the degradable material.

19. The method of claim 11, wherein the paranasal sinus or the opening to the paranasal sinus in which the biodegradable nasal support is positioned comprises a frontal sinus, an opening to the frontal sinus, a sphenoid sinus, an opening to the sphenoid sinus, an ethmoid sinus, an opening to the ethmoid sinus, a maxillary sinus, or an opening to the maxillary sinus of the patient.

20. The method of claim 11, wherein the interior lumen of the biodegradable nasal support has a length of from about 10 mm to about 20 mm and a diameter of from about 6 mm to about 10 mm.
